# EUROPEAN PATENT SPECIFICATION

(11) **EP 1 877 436 B1**
(45) Date of publication and mention of the grant of the patent: **13.01.2016**
(21) Application number: 05805917.1
(22) Date of filing: 29.07.2005
(51) Int. Cl.: A61K 48/00, A61K 38/00

(54) **USE OF MAPC OR PROGENY THEREFROM TO POPULATE LYMPHOHEMATOPOIETIC TISSUES**
VERWENDUNG VON MAPC ODER NACHKOMMEN DAVON ZUR BESIEDLUNG VON LYMPHOHÄMATOPOETISCHEN GEWEBEN
UTILISATION DE MAPC OU DE SA DESCENDANCE POUR PEUPLER DES TISSUS LYMPHO-HEMATOPOIETIQUES

(30) Priority: 05.05.2005 US 15740
(43) Date of publication of application: 16.01.2008
(73) Proprietor: Regents of the University of Minnesota, St. Paul, Minnesota 55114-8658 (US)
(72) Inventor: VERFAILLIE, Catherine M., White Bear Lake, Minnesota 55110 (US); LAKSHMIPATHY, Uma, Carlsbad, CA 92011 (US); SERAFINI, Marta, Biassono, MI 20046 (IT); BUCKLEY, Shannon, Minneapolis, MN 55408 (US)
(74) Representative: Lucas, Phillip Brian
(86) International application number: PCT/US2005/027147
(87) International publication number: WO 2006/121454

(56) References cited:
- EP-A- 1 491 093
- WO-A-01/11011
- WO-A-2004/050859
- WO-A-2006/047743
- WO-A2-02/057430
- WO-A2-02/064748
- GAMMAITONI LORETTA ET AL: "Elevated telomerase activity and minimal telomere loss in cord blood long-term cultures with extensive stem cell replication" BLOOD, vol. 103, no. 12, 15 June 2004 (2004-06-15), pages 4440-4448, XP002518562 ISSN: 0006-4971
- BENTZON J F ET AL: "Tissue distribution and engraftment of human mesenchymal stem cells immortalized by human telomerase reverse transcriptase gene" BIOCHEMICAL AND BIOPHYSICAL RESEARCH COMMUNICATIONS, ACADEMIC PRESS INC. ORLANDO, FL, US, vol. 330, no. 3, 22 March 2005 (2005-03-22), pages 633-640, XP004833686 ISSN: 0006-291X
- JIANG Y ET AL: "PLURIPOTENCY OF MESENCHYMAL STEM CELLS DERUVED FROM ADULT MARROW" NATURE, NATURE PUBLISHING GROUP, LONDON, UK, vol. 418, no. 6893, 4 July 2002 (2002-07-04), pages 41-49, XP001204372 ISSN: 0028-0836
- BARLOZZARI T ET AL: "INHIBITION OF PLURIPOTENT HEMATOPOIETIC STEM CELLS OF BONE MARROW BY LARGE GRANULAR LYMPHOCYTES" PROCEEDINGS OF THE NATIONAL ACADEMY OF SCIENCES OF THE UNITED STATES OF AMERICA, vol. 84, no. 21, 1987, pages 7691-7695, XP002518563 ISSN: 0027-8424
- REYES M VERFAILLIE C M: "Characterization of multipotent adult progenitor cells, a subpopulation of mesenchymal stem cells" ANNALS OF THE NEW YORK ACADEMY OF SCIENCES, NEW YORK ACADEMY OF SCIENCES, NEW YORK, NY, US, vol. 938, 1 January 2001 (2001-01-01), pages 231-235, XP002954850 ISSN: 0077-8923
- YOUNG ET AL.: 'Adult Stem Cells' ANAT. REC. PART A vol. 276A, 2004, pages 75 - 102, XP008117976
- VERFAILLIE C.M. ET AL.: 'Stem Cells: Hype and Reality' HEMATOLOGY 2002, pages 369 - 391, XP002383580

## Description

### Statement of Government Rights

This work was funded by Unites States Grant No. RO1 DK58295. The government may have certain rights to this invention.

### Field of the Invention

This invention relates to the field of non-embryonic stem cells, specifically to the use of multipotent adult stem cells (MAPCs) to provide lymphohematopoiesis and create functional immunity.

### Background of the Invention

### Hematopoiesis

During gastrulation, mesoderm is induced by the prospective endoderm and is patterned along the dorsal-ventral (dv) axis. Bone morphogenic proteins (BMPs) are important for specifying cells towards a ventral mesoderm fate (Hemmati-Brivanlou A and Thomsen 1995; Bhardwaj G et al. 2001; Leung AYH et al. 2004). In mammals, cells from ventral mesoderm migrate to the extra-embryonic yolk sac where they give rise to primitive hematopoiesis (Yoder M 1997). Primitive hematopoiesis is transient, consisting primarily of erythroid cells that express embryonic hemoglobin. Definitive hematopoiesis takes place in the aorto-gonad-mesonephros (AGM) region, where hematopoietic stem cells (HSCs) expand and migrate to the fetal liver and spleen to generate hematopoietic cells of all lineages. The major hematopoietic tissue post-natal is the bone marrow.

The role the bone marrow (BM) microenvironment plays in supporting self-renewing cell divisions of HSC has been studied. It has been demonstrated that *β*1-integrin mediated signaling controls self-renewal and differentiation of HSCs (Verfaillie C et al. 1991; Verfaillie C 1992; Lewis ID et al. 2001; Hurley RW et al. 1995; Jiang Y et al. 2000; Jiang Y et al. 2000) and a role for glycosaminoglycans as orchestrators of the HSC niche has been demonstrated (Lewis ID et al. 2001; Gupta P et al. 1996; Gupta P et al. 1998.).

### Stem Cells

The embryonic stem (ES) cell has unlimited self-renewal and can differentiate into all tissue types. ES cells are derived from the inner cell mass of the blastocyst or primordial germ cells from a post-implantation embryo (embryonic germ cells or EG cells). ES and EG cells have been derived from mouse, and, more recently, from non-human primates and humans. When introduced into blastocysts, ES cells can contribute to all tissues. A drawback to ES cell therapy is that, when transplanted in post-natal animals, ES and EG cells generate teratomas.

ES (and EG) cells can be identified by positive staining with antibodies to SSEA 1 (mouse) and SSEA 4 (human). At the molecular level, ES and EG cells express a number of transcription factors specific for these undifferentiated cells. These include Oct-4 and rex-1. Rex expression depends on Oct-4. Also found are the LIF-R (in mouse) and the transcription factors sox-2 and rox-1. Rox-1 and sox-2 are also expressed in non-ES cells. Another hallmark of ES cells is the presence of telomerase, which provides these cells with an unlimited self-renewal potential *in vitro.*

Oct-4 (Oct 3 in humans) is a transcription factor expressed in the pregastrulation embryo, early cleavage stage embryo, cells of the inner cell mass of the blastocyst, and embryonic carcinoma (EC) cells (Nichols J., et al 1998), and is down-regulated when cells are induced to differentiate. Expression of Oct-4 plays an important role in determining early steps in embryogenesis and differentiation. Oct-4, in combination with Rox-1, causes transcriptional activation of the Zn-finger protein Rex-1, also required for maintaining ES in an undifferentiated state ((Rosfjord and Rizzino A. 1997; Ben-Shushan E, et al. 1998). In addition, sox-2, expressed in ES/EC, but also in other more differentiated cells, is needed together with Oct-4 to retain the undifferentiated state of ES/EC (Uwanogho D et al. 1995). Maintenance of murine ES cells and primordial germ cells requires LIF.

The Oct-4 gene (Oct 3 in humans) is transcribed into at least two splice variants in humans, Oct 3A and Oct 3B. The Oct 3B splice variant is found in many differentiated cells whereas the Oct 3A splice variant (also previously designated Oct 3/4) is reported to be specific for the undifferentiated embryonic stem cell (Shimozaki et al. 2003).

Adult stem cells have been identified in most tissues. Hematopoietic stem cells are mesoderm-derived and have been purified based on cell surface markers and functional characteristics. The hematopoietic stem cell, isolated from bone marrow, blood, cord blood, fetal liver and yolk sac, is the progenitor cell that reinitiates hematopoiesis and generates multiple hematopoietic lineages. Hematopoietic stem cells can repopulate the erythroid, neutrophil-macrophage, megakaryocyte and lymphoid hematopoietic cell pool.

Jiang et al. (2002) disclose that when murine LacZ⁺ MAPCs are transplanted into sublethally irradiated NOD-SCID mice, they contribute to the hematopoietic system; however, with low hematopoietic engraftment levels, including no T-lymphoid cells. Additionally, Jiang et al. disclose that human MAPCs were not able to undergo hematopoietic differentiation *in vitro.*

### Hematopoietic Cell Transplantation

Hematopoietic cell transplantation has been utilized for over 30 years to treat malignant and non-malignant hematopoietic disorders (Thomas ED 1999). Although autologous bone marrow (BM) or peripheral blood (PB) grafts have been used to treat some malignancies, contaminating tumor cells often contribute to relapse. And while allogeneic grafts can treat several malignant disorders, many patients lack an appropriate HLA-matched donor, thereby excluding them from this therapy. Also, allografts are often associated with disabling and sometimes lethal graft versus host disease (GVHD; Howe CWS and Radde-Stepanick T. 1999).

Hence, the need persists to evaluate potential novel sources of cells for providing lymphohematopoiesis in a subject.

### Summary of the Invention

A population of non-embryonic stem cells, specifically, multipotent adult progenitor cells (MAPCs) can effectively provide lymphohematopoiesis. MAPCs can progressively differentiate *in vivo* where they can form lymphohematopoietic stem cells and progenitor cells that can mature into more mature lymphohematopoietic cell types. Alternatively, differentiated progeny of MAPCs, formed *ex vivo,* can be used to provide lymphohematopoiesis. They can be administered to a subject where they can further differentiate, if desired; or terminally-differentiated cells, formed from MAPCs *ex vivo,* can be administered.

MAPC is an acronym for "multipotent adult progenitor cell" (non-ES, non-EG, non-germ) that has the capacity to differentiate into cell types of more than one embryonic lineage. It can form cell types of all three primitive germ layers (ectodermal, endodermal and mesodermal). Genes found in ES cells are also found in MAPCs (e.g., telomerase, Oct 3/4, rex-1, rox-1, sox-2). Telomerase or Oct 3/4 can be recognized as genes that are primary products for the undifferentiated state. Telomerase is necessary for self renewal without replicative senescence.

One embodiment provides cells in a tissue of the lymphohematopoietic system comprising administering to a subject in need thereof an effective amount of MAPCs that is human non-embryonic stem (ES), non-germ and non-embryonic germ cells that are positive for one or more of telomerase, Oct-4, rex-1, sox-1 or SSEA-4, that have been isolated and/or cultured in the presence of an oxygen concentration less than 10% for use with Natural Killer cell function inhibitor in a method for the treatment of congenital or acquired lymphohematopoietic disorder by providing lymphohematopoietic cells of lymphoid, myeloid and erythroid lineage including T lymphoid cells, in a tissue of the lymphohematopoietic system.

The subject may have been exposed to radiation, chemotherapy or has a genetic deficiency (e.g., a shortage of a substance needed by the body due to a genetic abnormality), or the subject may have a congenital lymphohematopoietic disorder or an acquired malignant or nonmalignant lymphohematopoietic disorder. The disorder may comprise a leukemia, a myelodysplastic syndrome, a lymphoma, an inherited red blood cell abnormality, an anemia, an inherited platelet abnormality, an immune disorder, a lymphoproliferative disorder, a phagocyte disorder or a coagulation disorder. Alternatively the disorder may comprise chronic myelogenous leukemia (CML) or Fanconia anemia (FA).

The subject can be a mammal. The MAPCs or progeny therefrom can be autologous, allogeneic, xenogeneic or a combination thereof.

The MAPCs may differentiate into cells of one or more of the lymphoid lineage, myeloid lineage or crythroid lineage.

The tissue may be one or more of the subject's thymus, spleen, blood, bone marrow or lymph nodes.

One embodiment provides for the use of MAPCs to treat lymphohematopoictic disorders such as a leukemia, a myelodysplastic syndrome, a lymphoma, an inherited red blood cell abnormality, an anemia, an inherited platelet abnormality, an immune disorder, a lymphoproliferative disorder, a phagocyte disorder or a coagulation disorder.

Administered MAPCs or progeny may contribute to generation of lymphohematopoietic tissue by differentiating into cells of the spleen, thymus, lymph node, blood or bone marrow *in vivo.* Alternatively, or in addition, administered MAPCs or progeny may contribute to generation of lymphohematopoietic tissue by secreting cellular factors that aid in homing and recruitment of endogenous MAPCs or other stem cells, such as hematopoietic stem cells, or other more differentiated cells. Alternatively, or in addition, MAPCs or progeny may secrete factors that act on endogenous stem or progenitor cells causing them to differentiate, thereby enhancing function. Further, MAPCs or progeny may secrete factors that act on stem, progenitor, or differentiated cells, causing them to divide. Further, MAPCs or progeny may provide for angiogenesis or reduce or prevent apoptosis.

### Brief Description of the Drawings

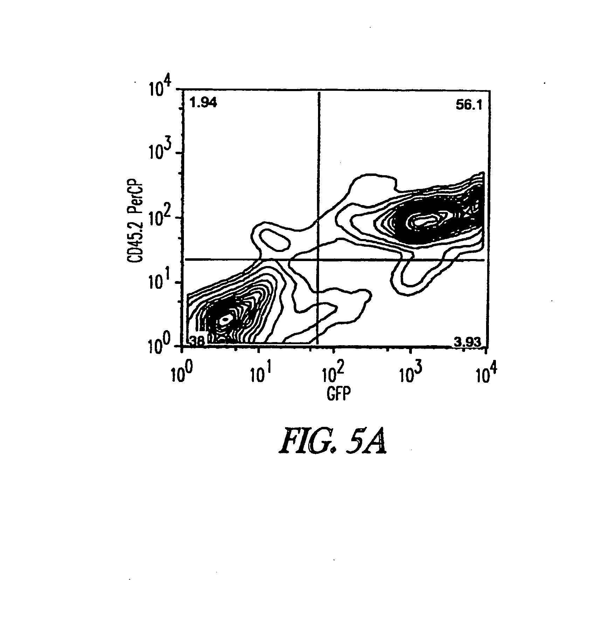

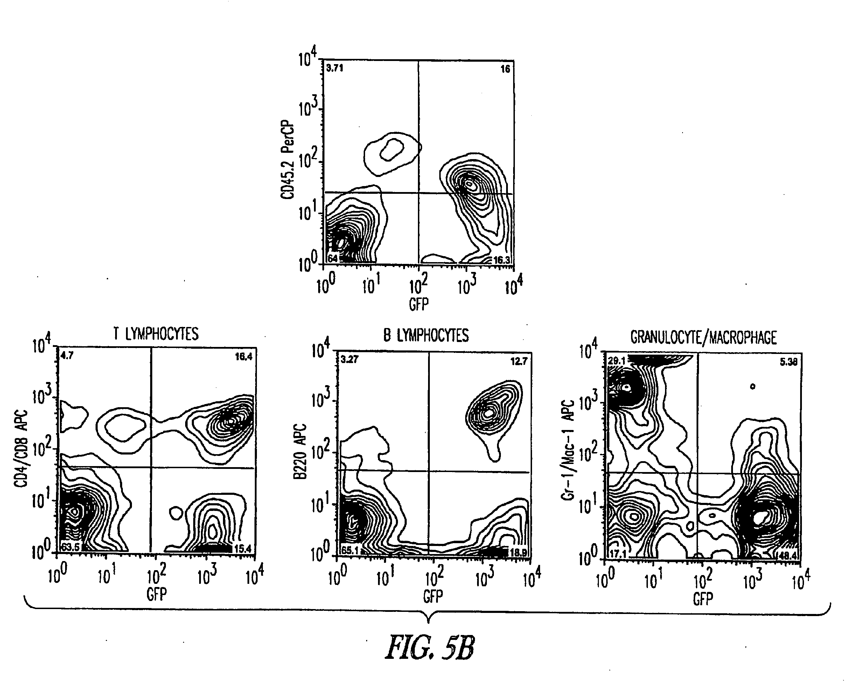

Figure 1 depicts hematopoietic stem cells and the structure of the hematopoietic compartment.

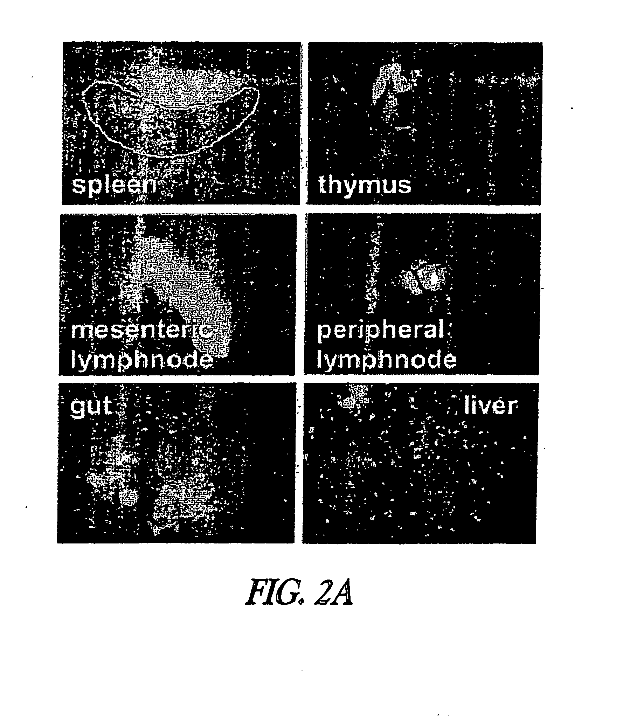

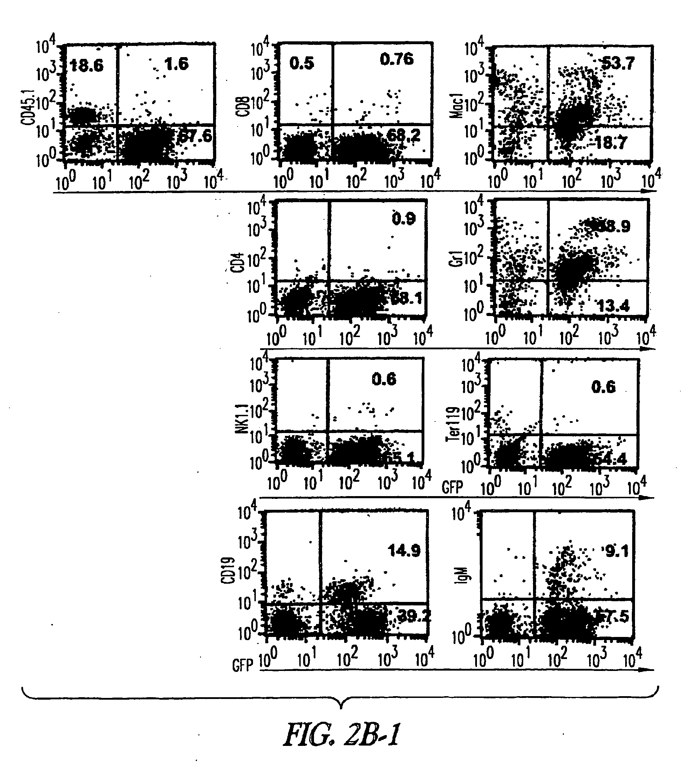

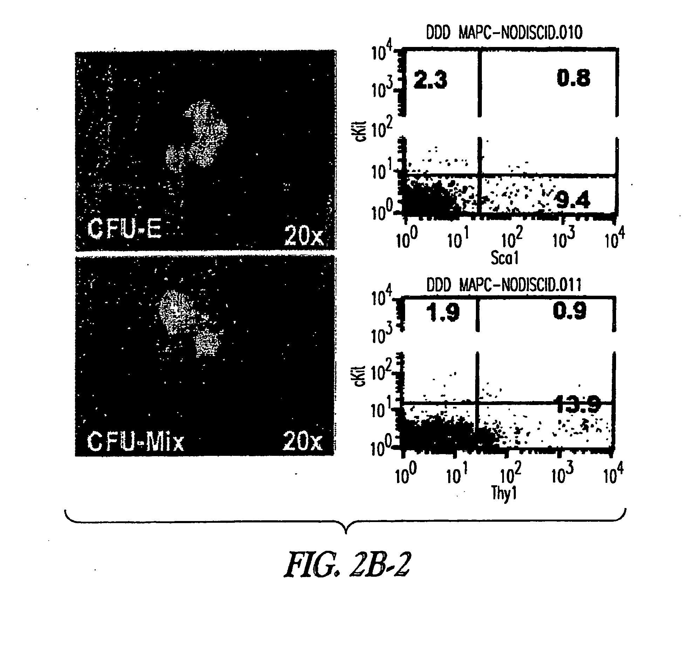

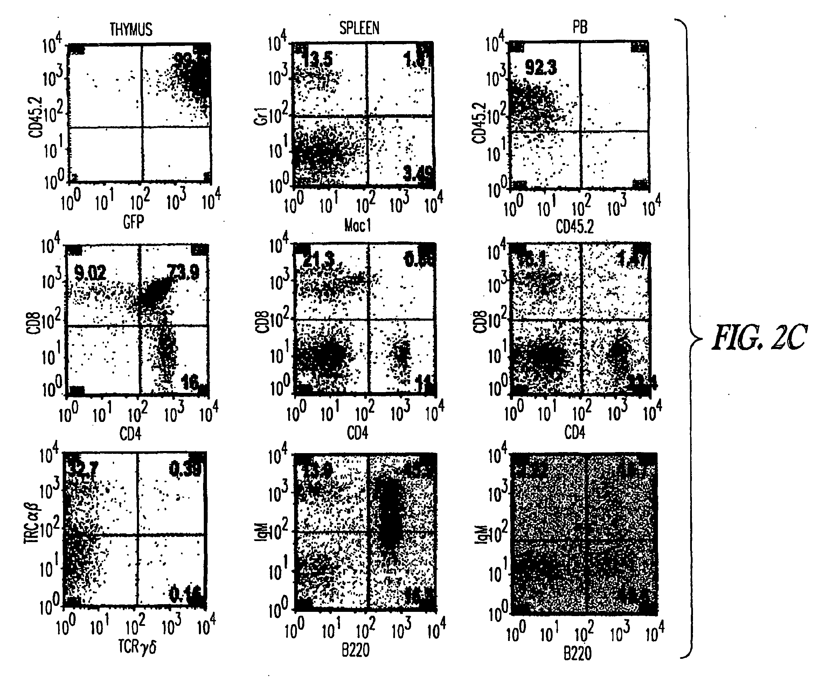

Figure 2 depicts lymphohematopoietic engraftment of MAPCs (A). 10⁶ GFP⁺ MAPC were transplanted in a NOD-SCID mouse (#6) treated with 275cGy and anti-NK antibody (anti-asialo-GM1). After 13 weeks, the animal was sacrificed and secondary lymphoid organs (spleen, mesenteric lymph nude, thymus and peripheral lymph node as well as gut and liver) were evaluated by intravital microscopy (using a Retiga camera mounted on a MZFLIII fluorescence stereomicroscope; images were captured with Q Imagin software). (B) Depicts hematopoietic cells from MAPCs in bone marrow (BM). Cells from bone marrow (BM) were evaluated by FACS. Data for cKit, Thyl and Sca1 in BM, represent gating on GFP positive cells only. Consistent with the notion that hematopoietic progenitors are generated (cKit, Sca1, Thy1⁺), Methocult culture of BM demonstrated GFP⁺CFU-E and CFU-Mix. (C) Depicts FACS analysis of thymus, spleen and peripheral blood (PB). Cells from thymus, PB and spleen were evaluated by FACS; except for the upper panel for thymus, the data represent gating on GFP positive cells only.
Figure 3 depicts *in vitro* hematopoiesis from mMAPCs. mMAPC were cocultured with EL08-1D2 cells with SCF, Tpo, IL3, IL6 for 2 weeks and subsequently in Methocult medium with SCF, IL3, IL6 and Epo. At d0, 14,29 and Q-RT-PCR was performed for hematopoietic (data not shown) and endothelial markers (A). (B) depicts BFU-E and CFU-Mix d15 in Methocult medium.

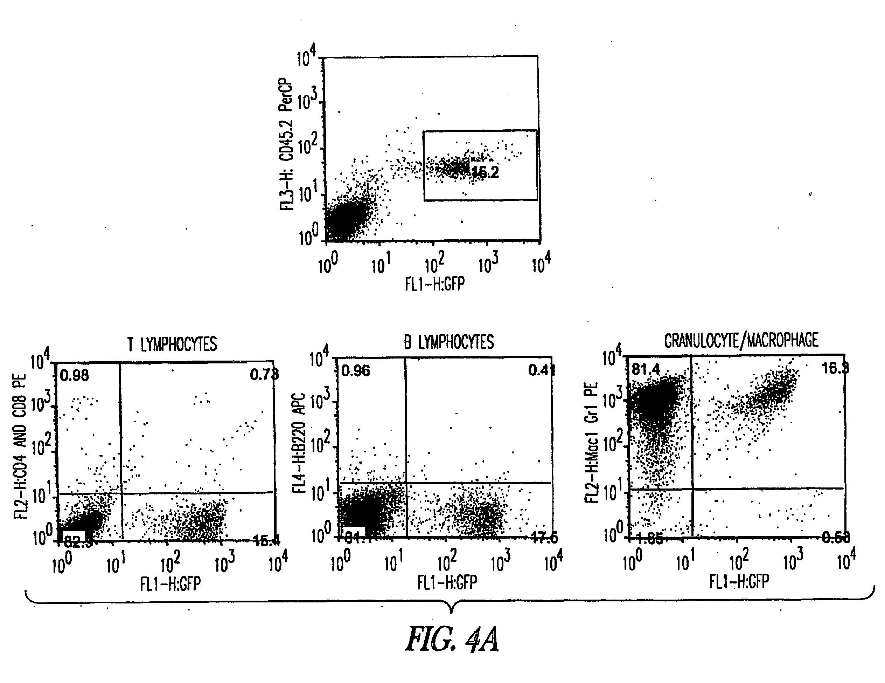

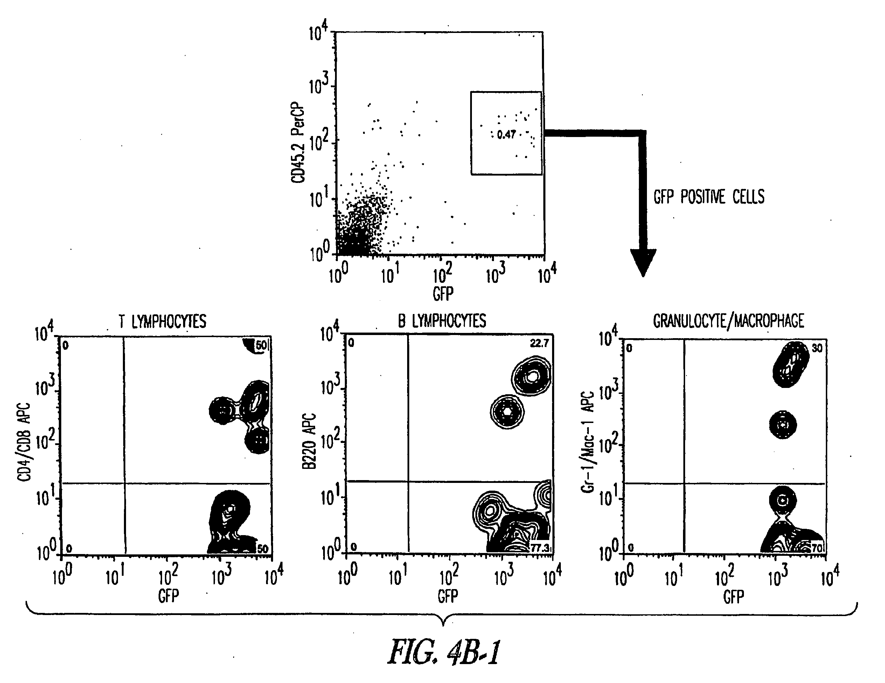

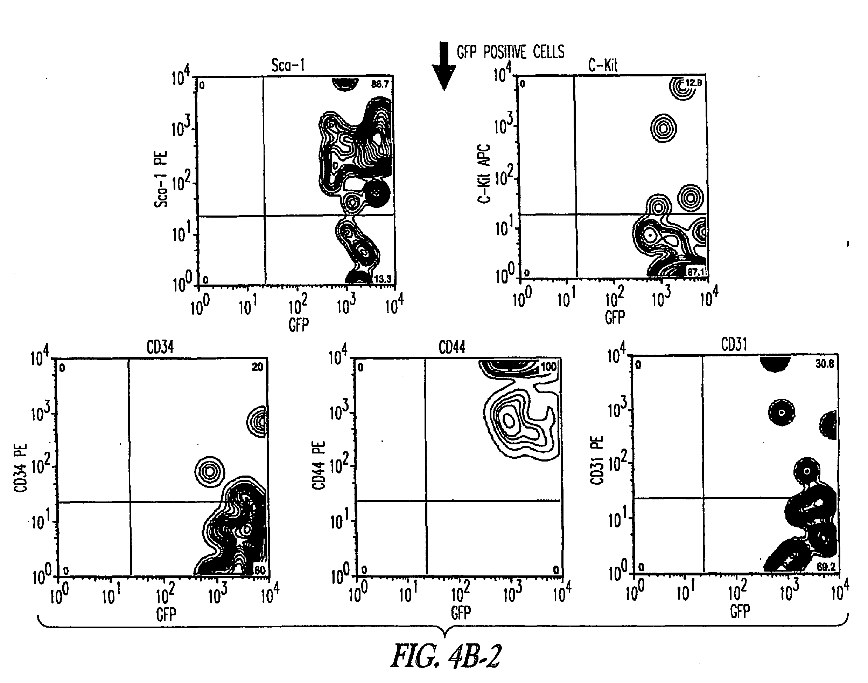

Figures 4A-B depict FACS analysis of the level of MAPC engraftment in mouse bone marrow.
) Figures 5A-B depict FACS analysis of the level of MAPC engraftment in mouse spleen.

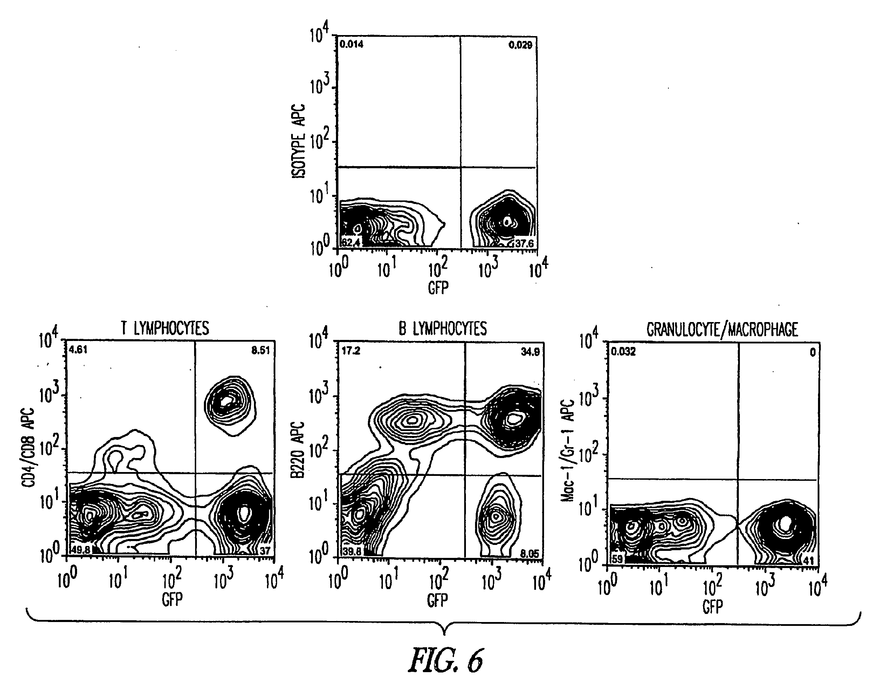

Figure 6 depicts FACS analysis of the level of MAPC engraftment in mouse peripheral blood.

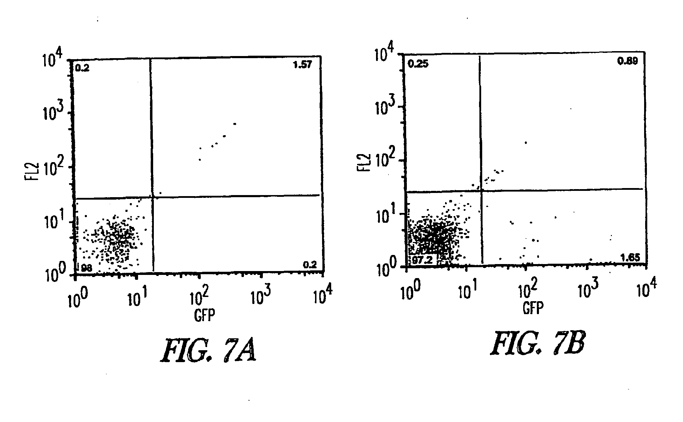

Figure 7 depicts FACS analysis of the level of engraftment in secondary mouse recipients (four weeks after the secondary transplant; secondary recipients were injected with full bone marrow (BM) derived from a mouse previously injected with MAPCs).

### Detailed Description of the Invention

MAPC have the ability to regenerate all primitive germ layers (endodermal, mesodermal and ectodermal) *in vitro* and *in vivo.* In this context they are equivalent to embryonic stem cells, and distinct from mesenchymal stem cells, which are also isolated from bone marrow. The biological potency of these cells has been proven in various animal models, including mouse, rat, and xenogeneic engraftment of human stem cells in rats or NOD/SCID mice (Reyes, M. and C.M. Verfaillie 2001; Jiang, Y. et al. 2002). Clonal potency of this cell population has been shown. Single genetically marked MAPC were injected into mouse blastocysts, blastocysts implanted, and embryos developed to term (Jiang, Y. et al. 2002). Post-natal analysis in chimeric animals showed reconstitution of all tissues and organs, including liver. Dual staining experiments demonstrated that gene-marked stem cells contributed to a significant percentage of apparently functional cardiomyocytes in these animals. These animals did not show any heart abnormalities or irregularities in either embryological or adult state. No abnormalities or organ dysfunction were observed in any of these animals.

### Definitions

As used herein, the terms below are defined by the following meanings:
"MAPC" is an acronym for "multipotent adult progenitor cell." It refers to a non-embryonic, non-germ, stem cell that can give rise to cell types of more than one embryonic lineage. It can form cell lineages of all three germ layers (i.e., endoderm, mesoderm and ectoderm) upon differentiation. Like embryonic stem cells, human MAPCs express telomerase, Oct 3/4 (i.e., Oct 3A), rex-1, rox-1 and sox-2 (Jiang, Y. et al. 2002). MAPCs derived from human, mouse, rat or other mammals appear to be the only normal, non-malignant, somatic cell (i.e., non-germ cell) known to date to express telomerase even in late passage cells. The telomeres are not sequentially reduced in length in MAPCs. MAPCs are karyotypically normal. MAPC may express SSEA-4 and nanog. The term "adult," with respect to MAPC is non-restrictive. It refers to a non-embryonic somatic cell.

Because MAPCs injected into a mammal can migrate to and assimilate within multiple organs, MAPCs are self-renewing stem cells. As such, they have utility in the repopulation of organs, either in a self-renewing state or in a differentiated state compatible with the organ of interest. They have the capacity to replace cell types that have been damaged, died, or otherwise have an abnormal function because of genetic or acquired disease. Or, as discussed below, they may contribute to preservation of healthy cells or production of new cells in a tissue.

"Multipotent," with respect to MAPC, refers to the ability to give rise to cell types of more than one embryonic lineage. MAPC can form cell lineages of all three primitive germ layers (i.e., endoderm, mesoderm and ectoderm) upon differentiation.

"Expansion" refers to the propagation of cells without differentiation.

"Progenitor cells" are cells produced during differentiation of a stem cell that have some, but not all, of the characteristics of their terminally-differentiated progeny. Defined progenitor cells, such as "hematopoietic progenitor cells," are committed to a lineage, but not to a specific or terminally-differentiated cell type. The term "progenitor" as used in the acronym "MAPC" does not limit these cells to a particular lineage.

"Self-renewal" refers to the ability to produce replicate daughter stem cells having differentiation potential that is identical to those from which they arose. A similar term used in this context is "proliferation."

"Engraft" or "engraftment" refers to the process of cellular contact and incorporation into an existing tissue of interest. In one embodiment, MAPCs or progeny derived therefrom engraft into the lymphohematopoietic system greater than about 10%, greater than about 15%, greater than about 20%, greater than about 25%, greater than about 30%, greater than about 35%, greater than about 40%, greater than about 45%, greater than about 50%, greater than about 55%, greater than about 60%, greater than about 65%, greater than about 70%, greater than about 75%, greater than about 80%, greater than about 85%, greater than about 90%, greater than about 95% or about 100%.

Persistence refers to the ability of cells to resist rejection and remain or increase in number over time (e.g., days, weeks, months, years) *in vivo.* Thus, by persisting, the MAPC or progeny can populate the tissues of the lymphohematopoietic system and reconstitute any deficient tissue.

"Immunologic tolerance" refers to the survival (in amount and/or length of time) of foreign (e.g., allogeneic or xenogeneic) tissues, organs or cells in recipient subjects. This survival is often a result of the inhibition of a graft recipient's ability to mount an immune response that would otherwise occur in response to the introduction of foreign cells. Immune tolerance can encompass durable immunosuppression of days, weeks, months or years. Included in the definition of immunologic tolerance is NK mediated immunologic tolerance.

The term "isolated" refers to a cell or cells which are not associated with one or more cells or one or more cellular components that are associated with the cell or cells *in vivo.*

An "enriched population" means a relative increase in numbers of MAPC relative to one or more non-MAPC cell types *in vivo* or in primary culture.

"Cytokines" refer to cellular factors that induce or enhance cellular movement, such as homing of MAPCs or other stem cells, progenitor cells or differentiated cells. Cytokines may also stimulate such cells to divide.

"Differentiation factors" refer to cellular factors, preferably growth factors or angiogenic factors, that induce lineage commitment.

A "subject" is a vertebrate, preferably a mammal, more preferably a human. Mammals include, but are not limited to, humans, farm animals, sport animals and pets.

As used herein, "treat," "treating" or "treatment" includes treating, preventing, ameliorating, or inhibiting an injury or disease related condition or a symptom of an injury or disease related condition.

An "effective amount" generally means an amount which provides the desired local or systemic effect, such as enhanced performance. For example, an effective dose is an amount sufficient to effect a beneficial or desired clinical result. The dose could be administered in one or more administrations and can include any preselected amount of cells. The precise determination of what would be considered an effective dose may be based on factors individual to each subject, including size, age, injury or disease being treated and amount of time since the injury occurred or the disease began. One skilled in the art, particularly a physician, would be able to determine the number of cells that would constitute an effective dose.

"Co-administer" can include simultaneous and/or sequential administration of two or more agents.

Administered MAPCs or progeny may contribute to generation of lymphohematopoietic tissue by differentiating into various cells in *vivo.* These cells may provide lymphohematopoiesis, engraft, repopulate, populate or reconstitute the various lymphohematopoietic tissues. Alternatively, or in addition, administered cells may contribute to generation of lymphohematopoietic tissue by secreting cellular factors that aid in homing and recruitment of endogenous MAPCs or other stem cells, or other more differentiated cells. Alternatively, or in addition, MAPCs or progeny may secrete factors that act on endogenous stem or progenitor cells causing them to differentiate. Further, MAPCs or progeny may secrete factors that act on stem, progenitor or differentiated cells, causing them to divide. Thus, MAPCs or progeny may provide benefit through trophic influences. Examples of trophic influences include, but are not limited to, improving cell survival and homing of cells to desired sites. Therapeutic benefit may be achieved by a combination of the above pathways.

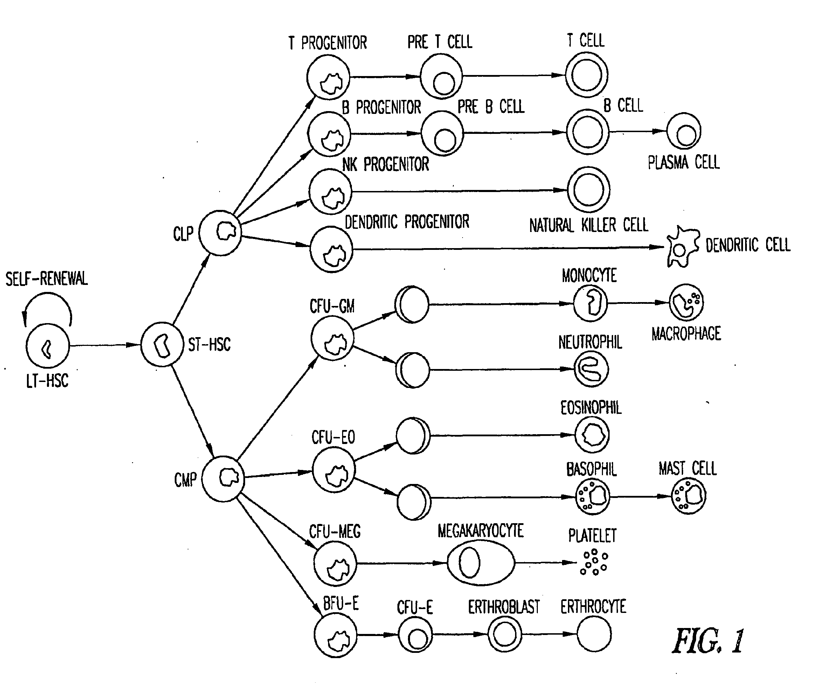

"Lymphohematopoiesis" refers to providing cells of blood, bone marrow, spleen, lymph nodes and thymus. These cells include those shown in Figure 1. It can involve proliferation of cells. It can also involve differentiation of cells. It can also involve recruitment of pre-existing cells to populate one or more tissues of the lymphohematopoietic system. It can also include reducing the rate or number of apoptotic cells in one or more tissues of the lymphohematopoietic system. Lymphohematopoietic cells include hematopoietic stem cells and cells from the lymphoid lineage, myoloid lineage, erythroid lineage, such as B cells, T cells, cells of the monocyte macrophage lineage, red blood cells, as well as such other cells which are derived from the hematopoietic stem cell (see, for example, Figure 1).

To provide lymphohematopoiesis in a subject, several routes arc possible. MAPCs can be administered and allowed to provide lymphohematopoiesis in *vivo.* This can occur, as described herein, by differentiation of the MAPCs themselves or by other means, such as by recruitment of endogenous colls. Alternatively, more mature cells can be administered, these cells having been differentiated *ex vivo* from MAPC. Such cells include progeny at all stages of differentiation, including hematopoietic stem cells that can give rise to all the mature hematopoietic cell types, committed progenitor cells that cannot form every one of those types, and further differentiated types, which can include fully mature lymphohematopoietic cells.

The terms "comprises", "comprising", and the like can have the meaning ascribed to them in U.S. Patent Law and can mean "includes", "including" and the like. As used herein, "including" or "includes" or the like means including, without limitation.

### MAPCs

Human MAPCs are described in U.S. Patent Application Serial Nos. 10/048,757 (PCT/US00/21387 (published as WO 01/11011)) and 10/467,963 (PCT/US02/04652 (published as WO 02/064748)). MAPCs have been identified in other mammals. Murine MAPCs, for example, are also described in PCT/US00/21387 (published as WO 01/11011) and PCT/US02/04652 (published as WO 02/064748). Rat MAPCs are also described in WO 02/064748.

Biologically and antigenically distinct from MSC, MAPC represents a more primitive progenitor cell population than MSC and demonstrates differentiation capability encompassing the epithelial, endothelial, neural, myogenic, hematopoietic, osteogenic, hepatogenic, chondrogenic and adipogenic lineages (Verfaillio, C.M. 2002; Jahagirdar, B.N. et al. 2001). MAPCs are capable of extensive culture without loss of differentiation potential and show efficient, long term, engraftment and differentiation along multiple developmental lineages in NOD-SCID mice, without evidence of teratoma formation (Reyes, M. and C.M. Verfaillie 2001).

Adherent cells from bone tissue are enriched in media as described herein, and grown to high population doublings. At early culture points more heterogeneity is detected in the population. Then, many adherent stromal cells undergo replicative senescence around cell doubling 30 and a more homogenous population of cells continues to expand and maintain long telomeres.

### Isolation and Growth

Methods ofMAPC isolation for humans and mouse arc described in the art. They are described in PCT/US00/21387 (published as WO 01/11011) and for rat in PCT/US02/04652 (published as WO 02/064748).

MAPCs were initially isolated from bone marrow, but were subsequently established from other tissues, including brain and muscle (Jiang, Y., et al., 2002). Thus, MAPCs can be isolated from multiple sources, including bone marrow, placenta, umbilical cord and cord blood, muscle, brain, liver, spinal cord, blood or skin. For example, MAPCs can be derived from bone marrow aspirates, which can be obtained by standard means available to those of skill in the art (see, for example, Muschler, G.F., et al., 1997; Batinic, D., et al., 1990). It is therefore now possible for one of skill in the art to obtain bone marrow aspirates, brain or liver biopsies and other organs, and isolate the cells using positive or negative selection techniques available to those of skill in the art, relying upon the genes that are expressed (or not expressed) in these cells (e.g., by functional or morphological assays, such as those disclosed in the above-referenced applications).

### MAPCs from Human Bone Marrow as Described in U.S. 10/048,757

Bone marrow mononuclear cells were derived from bone marrow aspirates, which were obtained by standard means available to those of skill in the art (see, for example, Muschler, G.F. et al. 1997; Batinic, D. et al. 1990). Multipotent adult stem cells are present within the bone marrow (or other organs such as liver or brain), but do not express the common leukocyte antigen CD45 or erythroblast specific glycophorin-A (Gly-A). The mixed population of cells was subjected to a Ficoll Hypaque separation. The cells were then subjected to negative selection using anti-CD45 and anti-Gly-A antibodies, depleting the population of CD45⁺ and Gly-A⁺ cells, and the remaining approximately 0.1% of marrow mononuclear cells were then recovered. Cells could also be plated in fibronectin-coatod wells and cultured as described below for 2-4 weeks to deplete the cell population of CD45⁺ and Gly-A⁺ cells.

Alternatively, positive selection can be used to isolate cells via a combination of cell-specific markers. Both positive and negative selection techniques are available to those of skill in the art, and numerous monoclonal and polyclonal antibodies suitable for negative selection purposes are also available in the art (see, for example, Leukocyte Typing V, Schlossman, et al., Eds. (1995) Oxford University Press) und are commercially available from a number of sources.

Techniques for mammalian cell separation from a mixture of cell populations have also been described by Schwartz, et al., in U. S. Patent No. 5,759,793 (magnetic separation), Basch et al. 1983 (immunoaffinity chromatography), and Wysocki and Sato 1978 (fluorescence-activated cell sorting).

Recovered CD45⁻/GlyA⁻ cells were plated onto culture dishes coated with about 5-115 ng/ml (about 7-10 ng/ml can be used) serum fibronectin or other appropriate matrix coating. Cells were maintained in Dulbecco's Minimal Essential Medium (DMEM) or other appropriate cell culture medium, supplemented with about 1-50 ng/ml (about 5-15 ng/ml can be used) platelet-derived growth factor-BB (PDGF-BB), about 1-50 ng/ml (about 5-15 ng/ml can be used) epidermal growth factor (EGF), about 1-50 ng/ml (about 5-15 ng/ml can be used) insulin-like growth factor (IGF), or about 100-10,000 IU (about 1,000 IU can be used) LIF, with about 10⁻¹⁰ to about 10⁻⁸ M dexamethasone or other appropriate steroid, about 2-10 µg/ml linoleic acid, and about 0.05-0.15 µM ascorbic acid. Other appropriate media include, for example, MCDB, MEM, IMDM and RPMI. Cells can either be maintained without serum, in the presence of about 1-2% fetal calf serum, or, for example, in about 1-2% human AB serum or autologous serum.

When re-seeded at about 2x10³ cells/cm² about every 3 days, >40 cell doublings were routinely obtained, and some populations underwent >70 cell doublings. Cell doubling time was about 36-48h for the initial 20-30 cell doublings. Afterwards cell-doubling time was extended to as much as 60-72h.

Telomere length of MAPCs from 5 donors (age about 2 years to about 55 years) cultured at re-seeding densities of about 2x10³ cells/cm² for about 23-26 cell doublings was between about 11-13 KB. This was about 3-5 KB longer than telomere length of blood lymphocytes obtained from the same donors. Telomere length of cells from 2 donors evaluated after about 23 and about 25 cell doublings, respectively, and again after about 35 cells doublings, was unchanged. The karyotype of these MAPCS was normal.

### Phenotype of Human MAPCs Under Conditions Described in U.S. 10/048,757

Immunophenotypic analysis by FACS of human MAPCs obtained after about 22-25 cell doublings showed that the cells do not express CD31, CD34, CD36, CD38, CD45, CD50, CD62E and -P, HLA-DR, Muc18, STRO-1, cKit, Tie/Tek; and express low levels of CD44, HLA-class I and *β*2-microglobulin, and express CD10, CD13, CD49b, CD49e, CDw90, Flkl (N>10).

Once cells underwent >40 doublings in cultures re-seeded at about 2x10³/cm², the phenotype became more homogenous and no cell expressed HLA class-I or CD44 (n=6). When cells were grown at higher confluence, they expressed high levels of Muc18, CD44, HLA class I and *β*2-microglobulin, which is similar to the phenotype described for MSC (N=8) (Pittenger, 1999).

Immunohistochemistry showed that human MAPCs grown at about 2x10³/cm² seeding density express EGF-R, TGF-R1 and -2, BMP-R1A, PDGF-R1a and -B, and that a small subpopulation (between about 1 and about 10%) of MAPCs stain with anti-SSEA4 antibodies (Kannagi, R 1983).

Using Clontech cDNA arrays the expressed gene profile of human MAPCs cultured at seeding densities of about 2x10³ cells/cm² for about 22 and about 26 cell doublings was determined:
A. MAPCs did not express CD31, CD36, CD62E, CD62P, CD44-H, cKit, Tie, receptors for IL1, IL3, IL6, IL11, G CSF, GM-CSF, Epo, Flt3-L, or CNTF, and low levels of HLA-class-I, CD44-E and Muc-18 mRNA.
B. MAPCs expressed mRNA for the cytokines BMP1, BMP5, VEGF, HGF, KGF, MCP1; the cytokine receptors Flkl, EGF-R, PDGF-R1α, gp130, LIF-R, activin-R1 and -R2, TGFR-2, BMP-R1A; the adhesion receptors CD49c, CD49d, CD29; and CD10.
C. MAPCs expressed mRNA for hTRT and TRF1; the POU domain transcription factor oct-4, sox-2 (required with oct-4 to maintain undifferentiated state of ES/EC, Uwanogho D. 1995), sox 11 (neural development), sox 9 (chondrogenesis) (Lefebvre V. 1998); homeodeomain transcription factors: Hoxa4 and -a5 (cervical and thoracic skeleton specification; organogenesis of respiratory tract) (Packer, A.I. 2000), Hox-a9 (myelopoiesis) (Lawrence, H. 1997), D1x4 (specification of forebrain and peripheral structures of head) (Akimenko, M.A. 1994), MSX1 (embryonic mesoderm, adult heart and muscle, chondro- and osteogenesis) (Foerst-Potts, L. 1997), PDX1 (pancreas) (Offield, M.F. 1996).
D. Presence of Oct-4, LIF-R, and hTRT mRNA was confirmed by RT-PCR.
E. In addition, RT-PCR showed that Rex-1 mRNA and Rox-1 mRNA were expressed in MAPCs.

Oct-4, Rex-1 and Rox-1 were expressed in MAPCs derived from human and murine marrow and from murine liver and brain. Human MAPCs expressed LIF-R and stained positive with SSEA-4. Finally, Oct-4, LIF-R, Rex-1 and Rox-1 mRNA levels were found to increase in human MAPCs cultured beyond 30 cell doublings, which resulted in phenotypically more homogenous cells. In contrast, MAPCs cultured at high density lost expression of these markers. This was associated with senescence before about 40 cell doublings and loss of differentiation to cells other than chondroblasts, osteoblasts and adipocytes.

### Culturing MAPCs as Described in U.S. 10/048,757

MAPCs isolated as described herein can be cultured using methods disclosed herein and in U.S. 10/048,757.

Briefly, for the culture of MAPCs, culture in low-serum or serum-free medium was preferred to maintain the cells in the undifferentiated state. Serum-free medium used to culture the cells, as described herein, was supplemented as described in Table 1. Human MAPCs do not require LIF.

**Table 1**

| | |
|---|---|
| Insulin | about 10 - 50 µg/ml (about 10 µg/ml)* |
| Transferrin | about 0 - 10 µg/ml (about 5.5 µg/ml) |
| Selenium | about 2 - 10 ng/ml (about 5 ng/ml) |
| Bovine serum albumin (BSA) | about 0.1 - 5 µg/ml (about 0.5 µg/ml) |
| Linoleic add | about 2 - 10 µg/ml (about 4.7 µg/ml) |
| Dexamethasone | about 0.005 - 0,15 µM (about 0.01 µM) |
| L-ascorbic acid 2-phosphate | about 0.1 mM |
| Low-glucose DMEM | about 40 - 60% (about 60%) |
| MCDB-201 | about 40 - 60% (about 40%) |
| Fetal calf serum | about 0-2% |
| Platelet-derived | about 5 - 15 ng (about 10 ng/ml) |
| Epidermal growth factor | about 5 - 15 ng/ml (about 10 ng/ml) |
| Insulin like growth factor | about 5 - 15 ng/ml (about 10 ng/ml) |
| Leukemia inhibitory factor | about 10-10,000IU (about 1,000 IU) |

| | |
|---|---|
| * Preferred concentrations are shown in parentheses. | |

Addition of about 10 ng/mL LIF to human MAPCs did not affect short-term cell growth (same cell doubling time till 25 cell doublings, level of Oct 4 (Oct 3/4) expression). In contrast to what was seen with human cells, when fresh murine marrow mononuclear cells, depleted on day 0 of CD45⁺ cells, were plated in MAPC culture, no growth was seen. When murine marrow mononuclear cells were plated, and cultured cells 14 days later depleted of CD45⁺ cells, cells with the morphology and phenotype similar to that of human MAPCs appeared. This suggested that factors secreted by hematopoiotic cells were needed to support initial growth of murine MAPCs. When cultured with PDGF-BB and EFG alone, cell doubling was slow (>6 days) and cultures could not be maintained beyond about 10 cell doublings. Addition of about 10 ng/mL LIF significantly enhanced cell growth.

Once established in culture, cells can be frozen and stored as frozen stocks, using DMEM with about 40% FCS and about 10% DMSO. Other methods for preparing frozen stocks for cultured cells are also available to those of skill in the art.

Thus, MAPCs could be maintained and expanded in culture medium that is available to the art. Such media include, but are not limited to Dulbecco's Modified Eagle's Medium® (DMEM), DMEM F12 medium®, Eagle's Minimum Essential Medium®, F-12K medium®, Iscove's Modified Dulbecco's Medium®, RPMI-1640 medium®. Many media are also available as a low-glucose formulation, with or without sodium pyruvate.

Also contemplated is supplementation of cell culture medium with mammalian sera. Sera often contain cellular factors and components that are necessary for viability and expansion. Examples of sera include fetal bovine serum (FBS), bovine serum (BS), calf serum (CS), fetal calf serum (FCS), newborn calf serum (NCS), goat serum (GS), horse serum (HS), human serum, chicken serum, porcine serum, sheep serum, rabbit serum, serum replacements, and bovine embryonic fluid. It is understood that sera can be heat-inactivated at about 55-65°C if deemed necessary to inactivate components of the complement cascade.

Additional supplements can also be used advantageously to supply the cells with the trace elements for optimal growth and expansion. Such supplements include insulin, transferrin, sodium selenium and combinations thereof. These components can be included in a salt solution such as, but not limited to Hanks' Balanced Salt Solution® (HBSS), Earle's Salt Solution®, antioxidant supplements, MCDB-201® supplements, phosphate buffered saline (PBS), ascorbic acid and ascorbic acid-2-phosphate, as well as additional amino acids. Many cell culture media already contain amino acids, however some require supplementation prior to culturing cells. Such amino acids include, but are not limited to L-alanine, L-arginine, L-aspartic acid, L-asparagine, L-cysteine, L-cystine, L-glutamic acid, L-glutamine, L-glycine, L-histidine, L-isoleucine, L-leucine, L-lysine, L-methionine, L-phenylalanine, L-proline, L-serine, L-threonine, L-tryptophan, L-tyrosine and L-valine. It is well within the skill of one in the art to determine the proper concentrations of these supplements.

Antibiotics are also typically used in cell culture to mitigate bacterial, mycoplasmal and fungal contamination. Typically, antibiotics or anti-mycotic compounds used are mixtures of penicillin/streptomycin, but can also include, but are not limited to amphotericin (Fungizone®), ampicillin, gentamicin, bleomycin, hygromycin, kanamycin, mitomycin, mycophenolic acid, nalidixic acid, neomycin, nystatin, paromomycin, polymyxin, puromycin, rifampicin, spectinomycin, tetracycline, tylosin and zeocin. Antibiotic and antimycotic additives can be of some concern, depending on the type of work being performed. One possible situation that can arise is an antibiotic-containing media wherein bacteria are still present in the culture, but the action of the antibiotic performs a bacteriostatic rather than bacteriocidal mechanism. Also, antibiotics can interfere with the metabolism of some cell types.

Hormones can also be advantageously used in cell culture and include, but are not limited to D-aldosterone, diethylstilbestrol (DES), dexamethasone, β-estradiol, hydrocortisone, insulin, prolactin, progesterone, somatostatin/human growth hormone (HGH), thyrotropin, thyroxine and L-thyronine.

Lipids and lipid carriers can also be used to supplement cell culture media, depending on the type of cell and the fate of the differentiated cell. Such lipids and carriers can include, but are not limited to cyclodextrin (α, β, y), cholesterol, linoleic acid conjugated to albumin, linoleic acid and oleic acid conjugated to albumin, unconjugated linoleic acid, linoleic-oleic-arachidonic acid conjugated to albumin, oleic acid unconjugated and conjugated to albumin, among others.

Also contemplated is the use of feeder cell layers. Feeder cells are used to support the growth of fastidious cultured cells, including stem cells. Feeder cells are normal cells that have been inactivated by γ-irradiation. In culture, the feeder layer serves as a basal layer for other cells and supplies cellular factors without further growth or division of their own (Lim, J.W. and Bodnar, A., 2002). Examples of feeder layer cells are typically human diploid lung cells, mouse embryonic fibroblasts, Swiss mouse embryonic fibroblasts, but can be any post-mitotic cell that is capable of supplying cellular components and factors that are advantageous in allowing optimal growth, viability and expansion of stem cells. In many cases, feeder cell layers are not necessary to keep the ES cells in an undifferentiated, proliferative state, as leukemia inhibitory factor (LIF) has anti-differentiation properties. Therefore, supplementation with LIF could be used to maintain MAPC in some species in an undifferentiated state.

Cells in culture can be maintained either in suspension or attached to a solid support, such as extracellular matrix components and synthetic or biopolymers. Stem cells often require additional factors that encourage their attachment to a solid support, such as type I, type II and type IV collagen, concanavalin A, chondroitin sulfate, fibronectin, "superfibronectin" and fibronectin-like polymers, gelatin, laminin, poly-D and poly-L-lysine, thrombospondin and vitronectin.

The maintenance conditions of stem cells can also contain cellular factors that allow stem cells, such as MAPCs, to remain in an undifferentiated form. It is advantageous under conditions where the cell must remain in an undifferentiated state of self-renewal for the medium to contain epidermal growth factor (EGF), platelet derived growth factor (PDGF), leukemia inhibitory factor (LIF; in selected species), and combinations thereof. It is apparent to those skilled in the art that supplements that allow the cell to self-renew but not differentiate should be removed from the culture medium prior to differentiation.

Stem cell lines and other cells can benefit from co-culturing with another cell type. Such co-culturing methods arise from the observation that certain cells can supply yet-unidentified cellular factors that allow the stem cell to differentiate into a specific lineage or cell type. These cellular factors can also induce expression of cell-surface receptors, some of which can be readily identified by monoclonal antibodies. Generally, cells for co-culturing are selected based on the type of lineage one skilled in the art wishes to induce, and it is within the capabilities of the skilled artisan to select the appropriate cells for co-culture.

MAPCs and lymphohematopoietic cells differentiated from MAPCs are useful as a source of cells for specific lymphohematopoietic lineages. The maturation, proliferation and differentiation of MAPCs may be effected through culturing MAPCs with appropriate factors including, but not limited to erythropoietin (EPO), colony stimulating factors, e.g., GM-CSF, G-CSF or M-CSF, SCF, interleukins, e.g., IL-1, -2, -3, -4, -5, -6, -7, -8, -13 etc., or with stromal cells or other cells which secrete factors responsible for stem cell regeneration, commitment and differentiation.

In one embodiment, human and mouse MAPCs can be differentiated into hematopoietic cells, such as hematopoietic stem or progenitor cells, *in vitro* as described herein below in Example 2. Methods for *in vitro* hematopoietic differentiation of MAPCs, including human, are also described in U.S. 10/048,757 (PCT/US00/21386, filed August 4, 2000) and U.S.10/467,963 (PCT/US02/04652, filed February 14, 2002).

Methods of identifying and subsequently separating differentiated cells from their undifferentiated counterparts can be carried out by methods well known in the art. Cells that have been induced to differentiate can be identified by selectively culturing cells under conditions whereby differentiated cells outnumber undifferentiated cells. Similarly, differentiated cells can be identified by morphological changes and characteristics that are not present on their undifferentiated counterparts, such as cell size, the number of cellular processes (i.e. formation of dendrites or branches), and the complexity of intracellular organelle distribution. Also contemplated are methods of identifying differentiated cells by their expression of specific cell-surface markers such as cellular receptors and transmembrane proteins. Monoclonal antibodies against these cell-surface markers can be used to identify differentiated cells. Detection of these cells can be achieved through fluorescence activated cell sorting (FACS) and enzyme-linked immunosorbent assay (BLISA). From the standpoint of transcriptional upregulation of specific genes, differentiated cells often display levels of gene expression that are different from undifferentiated cells. Reverse-transcription polymerase chain reaction (RT-PCR) can also be used to monitor changes in gene expression in response to differentiation. In addition, whole genome analysis using microarray technology can be used to identify differentiated cells.

Accordingly, once differentiated cells are identified, they can be separated from their undifferentiated counterparts, if necessary. The methods of identification detailed above also provide methods of separation, such as FACS, preferential cell culture methods, ELISA, magnetic beads, and combinations thereof. A preferred embodiment of the invention envisions the use of FACS to identify and separate cells based on cell-surface antigen expression.

### Additional Culture Methods

In additional experiments it has been found that the density at which MAPCs are cultured can vary from about 100 cells/cm² or about 150 cells/cm² to about 10,000 cells/cm², including about 200 cells/cm² to about 1500 cells/cm² to about 2000 cells/cm². The density can vary between species. Additionally, optimal density can vary depending on culture conditions and source of cells. It is within the skill of the ordinary artisan to determine the optimal density for a given set of culture conditions and cells.

Also, effective atmospheric oxygen concentrations of less than about 10%, including about 3 - 5%, can be used at any time during the isolation, growth and differentiation of MAPCs in culture.

### Uses of MAPCs and Progeny Therefrom in the Lymphohematopoietic System

MAPCs may be an alternative source of HSCs to treat congenital or acquired lymphohematopoietic disorders, or to establish chimerism prior to using the same cells or differentiated progeny therefrom to treat disorders amenable to MAPC-derived therapies. MAPCs have capacity for lymphohematopoietic differentiation both *in vivo* and *in vitro.* As discussed below in the Examples, when murine MAPCs were cocultured with E11.5 embryonic liver feeder cells, EL08-1D2, in the presence of cytokines for about 2 weeks, followed by culture in a colony-forming cell (CFC) assay, erythroid burst-forming unit (BFU-E) and mixed colony forming unit (CFU-Mix) colonies were detected. When murine GFP transgenic MAPCs were transplanted into NOD-SCID mice irradiated at about 275cGy and treated with an anti-NK antibody, up to about 90% GFP⁺CD45⁺ cells were detected in bone marrow (BM) at about 20 weeks, with differentiation to myeloid, B- and T-lymphoid cells. Likewise, when human MAPCs were transplanted into NOD-SCID mice, lymphohematopoietic engraftment was seen. Thus, HSCs can be generated from murine as well as human MAPCs, *in vivo* and *in vitro.* This process can used to radioprotect as well as provide long term population of the lymphohematopoietic system.

Hence, MAPCs are an attractive source of stem cells to treat lymphohematopoietic disorders including, but not limited to, congenital and acquired malignant and non-malignant lymphohematopoietic disorders. Compositions and methods of the invention are directed to the formation and use of MAPCs and progeny thereof to provide lymphohematopoietic cells to the various lymphohematopoietic tissues. In one embodiment, MAPCs or progeny therefrom are used for the treatment of lymphohematopoietic disorders. "Lymphoematopoietic disorders" refers to any disease or disorder of the lymphohematopoietic system. This system includes spleen, thymus, lymph node, blood (e.g., peripheral blood; PB) and bone marrow (BM).

In one embodiment, lymphohematopoietic disorders include, but are not limited to:
- leukemias (leukemia is a cancer of the immune system, whose cells are called leukocytes or white cells) including but not limited to Acute Leukemia, Acute Lymphoblastic Leukemia (ALL), Acute Myelogenous Leukemia (AML), Acute Biphenotypic Leukemia, Acute Undifferentiated Leukemia, Chronic Leukemia, Chronic Myelogenous Leukemia (CML), Chronic Lymphocytic Leukemia (CLL), Juvenile Chronic Myelogenous Leukemia (JCML), Juvenile Myelomonocytic Leukemia (JMML);
- myelodysplastic syndromes (myelodysplasia is sometimes called pre-leukemia) including but not limited to Refractory Anemia (RA), Refractory Anemia with Ringed Sideroblasts (RARS), Refractory Anemia with Excess Blasts (RAEB), Refractory Anemia with Excess Blasts in Transformation (RAEB-T), Chronic Myelomonocytic Leukemia (CMML);
- lymphomas (lymphoma is a cancer of the leukocytes that circulate in the blood and lymph vessels) including but not limited to Hodgkin's Lymphoma, Non-Hodgkin's Lymphoma, Burkitt's Lymphoma;
- inherited red cell (Erythrocyte) abnormalities (red cells contain hemoglobin and carry oxygen to the body) including but not limited to Beta Thalassemia Major (also known as Cooley's Anemia), Blackfan-Diamond Anemia, Pure Red Cell Aplasia, Sickle Cell Disease;
- other disorders of blood cell proliferation including but not limited to anemias (anemias are deficiencies or malformations of red cells) including but not limited to severe Aplastic Anemia, Congenital Dyserythropoietic Anemia, and Fanconi Anemia, recovery from anemia induced by accidental radiation exposure or induced by radiation or chemotherapeutic conditioning for bone marrow transplant in an oncology setting, Paroxysmal Nocturnal Hemoglobinuria (PNH),
- inherited platelet abnormalities (platelets are small blood cells needed for clotting) including but not limited to Amegakaryocytosis/Congenital Thrombocytopenia, Glanzmann Thrombasthenia, Myeloproliferative Disorders, Acute Myelofibrosis, Agnogenic Myeloid Metaplasia (Myelofibrosis), Polycythemia Vera, Essential Thrombocythemia;
- inherited immune system disorders including but not limited to Severe Combined Immunodeficiency (SCID) including but not limited to SCID with Adenosine Deaminase Deficiency (ADA-SCID), SCID which is X-linked, SCID with absence of T & B Cells, SCID with absence of T Cells, Normal B Cells, Omenn Syndrome, Neutropenias including but not limited to Kostmann Syndrome, Myelokathexis; Ataxia-Telangiectasia, Bare Lymphocyte Syndrome, Common Variable Immunodeficiency, DiGeorge Syndrome, Leukocyte Adhesion Deficiency;
- lymphoproliferative disorders (LPD) including but not limited to Lymphoproliferative Disorder, X-linked (also known as Epstein-Barr Virus Susceptibility), Wiskott-Aldrich Syndrome;
- phagocyte disorders (phagocytes are immune system cells that can engulf and kill foreign organisms) including but not limited to Chediak-Higashi Syndrome, Chronic Granulomatous Disease, Neutrophil Actin Deficiency, Reticular Dysgenesis; and
- cancers in the bone marrow (plasma cell disorders) including but not limited Multiple Myeloma, Plasma Cell Leukemia, Waldenstrom's Macroglobulinemia.

Other examples of lymphohematological diseases which can be treated using MAPCs or progeny derived therefrom include, but are not limited to, graft versus host disease (GVHD), autoimmune diseases, coagulation disorders/coagulation factor deficiencies, such as hemophilia, thalassemia, chronic granulomatous disease and lysosomal storage diseases/enzyme deficiencies, such as Gaucher disease. MAPCs may also be used in the production of a chimeric immune system allowing host acceptance of donor organ or tissue graft (tolerance), for example, islet transplant, heart or kidney transplant. MAPCs also have use in the production of donor or chimeric immune system allowing for repair of inherited genetic deficiencies, such as sickle cell anemia. Furthermore, MAPCS may be used for the replacement of host immune system for treatment of autoimmune disease, such as lupus, myasthenia gravis, multiple sclerosis, rheumatoid arthritis or diabetes.

For example, with the use of MAPC therapy, one can restore, partially or completely, lymphohematopoiesis from well before the HSC state. Thus, use of MAPCs may provide better treatment outcomes than CD34⁺ transplants currently in use now for autoimmune disorders since the newly educated T cells would not be autoimmune. Therefore, one could use MAPCs prior to the formation of the CD34⁺ cell state, and thus, produce newly formed T cells.

MAPCs, or their differentiated progeny, have use in gene therapy. Expression vectors may be introduced into and expressed in autologous, allogeneic or xenogeneic MAPCs, or their differentiated progeny, or the genome of the cells may be modified by homologous or non-homologous recombination by methods known in the art. In this way, one may correct genetic defects in an individual. For example, diseases including, but not limited to, *β*-thalassemia, sickle cell anemia, adenosine deaminase deficiency or recombinase deficiency may be corrected.

Additionally, one may express in MAPCs, or their differentiated progeny, a ribozyme, antisense RNA or protein to inhibit the expression or activity of a particular gene product. Drug resistance genes including, but not limited to, the multiple drug resistant (MDR) gene, may also be introduced into MAPCs, or their differentiated progeny, to enable them to survive drug therapy. For lymphohematotrophic pathogens, such as HIV or HTLV-I and HTLV-II, the MAPCs, or their differentiated progeny, can be genetically modified to produce an antisense RNA, ribozyme or protein which would prevent proliferation of a pathogen in MAPCs, or their differentiated progeny. One may also disable or modulate the expression of a particular genetic sequence by methods known in the art, including, but not limited to, directly substituting, deleting or adding DNA by non-homologous or homologous recombination or, indirectly, by antisense sequences.

The MAPCs, or their differentiated progeny, may be employed as grafts for bone marrow transplantation to treat malignancies, bone marrow failure states and congenital metabolic, immunologic or lymphohematologic disorders. For example, marrow samples can be obtained from the subject and MAPCs isolated. The MAPCs can then be expanded *in vitro* and serve as a graft for autologous marrow transplantation. Alternatively, the MAPCs can be differentiated to lymhohematopoietic cells *ex vivo* prior to transplantation. The MAPCs, or their differentiated progeny, can also be genetically manipulated prior to transplantation. The cells are generally infused after the subject has received curative chemo-radiotherapy.

The expanded cells can also be utilized for *in utero* transplantation during the first trimester of pregnancy. Fetuses with metabolic or lymphohematologic disorders can be diagnosed prenatally. Marrow may be obtained from normal individuals and MAPCs can be obtained and expanded *in vitro.* The MAPCs can then be administered to the fetus by *in utero* injection, for example. Alternatively, the MAPCs can be differentiated to lymphohematopoietic cells prior to transplantation. The MAPCs, or their differentiated progeny, can also be genetically manipulated prior to transplantation. Thus, a chimera will be formed which will lead to full or partial alleviation of the clinical abnormality.

Lymphohematopoiesis can be detected by any means available to one of skill in the art. There are many tests available to one of skill in the art to determine/test blood function. For example, a CBC (Complete Blood Count) is a common blood test that provides detailed information about three types of cells in blood: red blood cells, white blood cells and platelets. Additionally, one may use fluorescence activated cell sorting (FACS). FACS analysis can be performed to detect any population of hematopoietic cells (e.g., T cells, B cells, granulocytes, macrophages, an immature population of T or B cells, red blood cells). A CFU-S assay may also be used. One may also measure red blood cells or a parameter thereof, such as a test to measure hemoglobin concentration, red blood cell count or red blood cell half-life. Lymphohematopoiesis in a treated subject can be compared with a control value of lymphohematopoiesis. In one embodiment, the control value is obtained from a normal subject (a subject not in need of treatment). In another embodiment, the control value is obtained from the subject prior to treatment, or at time intervals after treatment.

MAPCs also find use in the establishment of a human immune system in an animal (e.g., an immunodeficient mouse) which has uses including but not limited to: 1) screening for agents (e.g., biologicals or small molecules) which regulate human lymphohematopoiesis; 2) screening to test potenial antigens for human vaccine production; 3) production of human antibodies (by antigenic challenge of animal) for humoral immunity or treatment of infectious disease; or 4) production of antigen specific T cells with cytotoxic, helper or regulatory properties.

### Administration of MAPCs

MAPCs, or their differentiated progeny, can be administered to a subject by a variety of methods available to the art, including but not limited to localized injection, catheter administration, systemic injection, intraperitoneal injection, parenteral administration, oral administration, intracranial injection, intra-arterial injection, intravenous injection, intraventricular infusion, intraplacental injection, intrauterine injection, surgical intramyocardial injection, transendocardial injection, transvascular injection, intracoronary injection, transvascular injection, intramuscular injection, surgical injection into a tissue of interest or via direct application to tissue surfaces (e.g., during surgery or on a wound).

MAPCs can be administered either peripherally or locally through the circulatory system. "Homing" of stem cells would concentrate the implanted cells in an environment favorable to their growth and function. Pre-treatment of a patient with cytokine(s) to promote homing is another alternative contemplated in the methods of the present invention. Certain cytokines (e.g., cellular factors that induce or enhance cellular movement, such as homing of MAPCs or other stem cells, progenitor cells or differentiated cells) can enhance the migration of MAPCs. Cytokines include, but are not limited to, stromal cell derived factor-1 (SDF-1), stem cell factor (SCF), angiopoietin-1, placenta-derived growth factor (PIGF) and granulocyte-colony stimulating factor (G-CSF). Cytokines also include any which promote the expression of endothelial adhesion molecules, such as ICAMs, VCAMs and others, which facilitate the homing process.

Differentiation of MAPCs to a phenotype characteristic of a desired tissue can be enhanced when differentiation factors are employed, e.g., factors promoting lymphohematopoietic cell formation.

Viability of newly forming tissues can be enhanced by angiogenesis. Factors promoting angiogenesis include but are not limited to VEGF, aFGF, angiogenin, angiotensin-1 and -2, betacellulin, bFGF, Factor X and Xa, HB-EGF, PDGF, angiomodulin, angiotropin, angiopoetin-1, prostaglandin E1 and E2, steroids, heparin, 1-butyryl-glycerol and nicotinic amide.

Factors that decrease apoptosis can also promote the formation of new tissue, such as lymphohematopoietic tissues. Factors that decrease apoptosis include but are not limited to β-blockers, angiotensin-converting enzyme inhibitors (ACE inhibitors), AKT, HIF, carvedilol, angiotensin II type 1 receptor antagonists, caspase inhibitors, cariporide and eniporide.

In one embodiment, one or more factors which promote lymphohematopoiesis, such as a biological, including EPO, or a small molecule, is administered prior to, after or concomitantly with MAPCs or their differentiated progeny.

Exogenous factors (e.g., cytokines, differentiation factors (e.g., cellular factors, such as growth factors or angiogenic factors that induce lineage commitment), angiogenesis factors and anti-apoptosis factors) can be administered prior to, after or concomitantly with MAPCs or their differentiated progeny. For example, a form of concomitant administration would comprise combining a factor of interest in the MAPC suspension media prior to administration. Administrations are variable and may include an initial administration followed by subsequent administrations.

A method to potentially increase cell survival is to incorporate MAPCs or progeny into a biopolymer or synthetic polymer. Depending on the patient's condition, the site of injection might prove inhospitable for cell seeding and growth because of scarring or other impediments. Examples of biopolymer include, but are not limited to, fibronectin, fibrin, fibrinogen, thrombin, collagen and proteoglycans. This could be constructed with or without included cytokines, differentiation factors, angiogenesis factors or anti-apoptosis factors. Additionally, these could be in suspension. Another alternative is a three-dimensional gel with cells entrapped within the interstices of the cell biopolymer admixture. Again cytokines, differentiation factors, angiogenesis factors anti-apoptosis factors or a combination thereof could be included within the gel. These could be deployed by injection via various routes described herein.

In current human studies of autologous mononuclear bone marrow cells, empirical doses ranging from about 1 to 4 x 10⁷ cells have been used. However, different scenarios may require optimization of the amount of cells administered. Thus, the quantity of cells to be administered will vary for the subject being treated. In a preferred embodiment, between about 10⁴ to 10⁸, more preferably about 10⁵ to 10⁷ and most preferably, about 3 x 10⁷ stem cells and optionally, about 50 to 500 µg/kg per day of a cytokine can be administered to a human subject. However, the precise determination of what would be considered an effective dose may be based on factors individual to each patient, including their size, age, disease or injury, amount of damage, amount of time since the damage occurred and factors associated with the mode of delivery (direct injection - lower doses, intravenous - higher doses). Dosages can be readily ascertained by those skilled in the art from this disclosure and the knowledge in the art.

An issue regarding the use of stem cells is the purity of the isolated stem cell population. Bone marrow cells, for example, comprise mixed populations of cells, which can be purified to a degree sufficient to produce a desired effect. Those skilled in the art can readily determine the percentage of MAPCs in a population using various well-known methods, such as fluorescence activated cell sorting (FACS). Preferable ranges of purity in populations comprising MAPCs, or their differentiated progeny, are about 50-55%, about 55-60%, and about 65-70%. More preferably the purity is about 70-75%, about 75-80%, about 80-85%; and most preferably the purity is about 85-90%, about 90-95%, and about 95-100%. However, populations with lower purity can also be useful, such as about 25-30%, about 30-35%, about 35-40%, about 40-45% and about 45-50%. Purity of MAPCs can be determined according to the gene expression profile within a population. Dosages can be readily adjusted by those skilled in the art (e.g., a decrease in purity may require an increase in dosage).

The skilled artisan can readily determine the amount of cells and optional additives, vehicles, or carrier in compositions to be administered in methods of the invention. Typically, additives (in addition to the active stem cell(s) or cytokine(s)) are present in an amount of 0.001 to 50 wt % solution in phosphate buffered saline, and the active ingredient is present in the order of micrograms to milligrams, such as about 0.0001 to about 5 wt %, preferably about 0.0001 to about 1 wt %, most preferably about 0.0001 to about 0.05 wt % or about 0.001 to about 20 wt %, preferably about 0.01 to about 10 wt %, and most preferably about 0.05 to about 5 wt %. Of course, for any composition to be administered to an animal or human, and for any particular method of administration, it is preferred to determine therefore: toxicity, such as by determining the lethal dose (LD) and LD₅₀ in a suitable animal model e.g., a rodent, such as mouse; and, the dosage of the composition(s), concentration of components therein and timing of administering the composition(s), which elicit a suitable response. Such determinations do not require undue experimentation from the knowledge of the skilled artisan, this disclosure and the documents cited herein. And, the time for sequential administrations can be ascertained without undue experimentation.

When administering a therapeutic composition of the present invention, it will generally be formulated in a unit dosage injectable form (solution, suspension, emulsion). The pharmaceutical formulations suitable for injection include sterile aqueous solutions and dispersions. The carrier can be a solvent or dispersing medium containing, for example, water, saline, phosphate buffered saline, polyol (for example, glycerol, propylene glycol, liquid polyethylene glycol, and the like) and suitable mixtures thereof.

Additionally, various additives which enhance the stability, sterility, and isotonicity of the compositions, including antimicrobial preservatives, antioxidants, chelating agents and buffers, can be added. Prevention of the action of microorganisms can be ensured by various antibacterial and antifungal agents, for example, parabens, chlorobutanol, phenol, sorbic acid, and the like. In many cases, it will be desirable to include isotonic agents, for example, sugars, sodium chloride, and the like. Prolonged absorption of the injectable pharmaceutical form can be brought about by the use of agents delaying absorption, for example, aluminum monostearate and gelatin. According to the present invention, however, any vehicle, diluent, or additive used would have to be compatible with the cells.

Sterile injectable solutions can be prepared by incorporating the cells utilized in practicing the present invention in the required amount of the appropriate solvent with various amounts of the other ingredients, as desired.

In one embodiment, MAPCs, or differentiated progeny thereof, can be administered initially, and thereafter maintained by further administration of MAPCs or differentiated progeny thereof. For instance, MAPCs can be administered by one method of injection, and thereafter further administered by a different or the same type of method.

It is noted that human subjects are treated generally longer than canines or other experimental animals, such that treatment has a length proportional to the length of the disease process and effectiveness. The doses may be single doses or multiple doses over a period of several days. Thus, one of skill in the art can scale up from animal experiments, e.g., rats, mice, canines and the like, to humans, by techniques from this disclosure and documents cited herein and the knowledge in the art, without undue experimentation. The treatment generally has a length proportional to the length of the disease process and drug effectiveness and the subject being treated.

Examples of compositions comprising MAPCs, or differentiated progeny thereof, include liquid preparations for administration, including suspensions, and, preparations for direct or intravenous administration (e.g., injectable administration), such as sterile suspensions or emulsions. Such compositions may be in admixture with a suitable carrier, diluent, or excipient such as sterile water, physiological saline, glucose, dextrose, or the like. The compositions can also be lyophilized. The compositions can contain auxiliary substances such as wetting or emulsifying agents, pH buffering agents, gelling or viscosity enhancing additives, preservatives, flavoring agents, colors, and the like, depending upon the route of administration and the preparation desired. Standard texts, such as "REMINGTON'S PHARMACEUTICAL SCIENCE," 17th edition, 1985, may be consulted to prepare suitable preparations, without undue experimentation.

Compositions of the invention are conveniently provided as liquid preparations, e.g., isotonic aqueous solutions, suspensions, emulsions or viscous compositions, which may be buffered to a selected pH. Liquid preparations are normally easier to prepare than gels, other viscous compositions and solid compositions. Additionally, liquid compositions are somewhat more convenient to administer, especially by injection. Viscous compositions, on the other hand, can be formulated within the appropriate viscosity range to provide longer contact periods with specific tissues.

The choice of suitable carriers and other additives will depend on the exact route of administration and the nature of the particular dosage form, e.g., liquid dosage form (e.g., whether the composition is to be formulated into a solution, a suspension, gel or another liquid form, such as a time release form or liquid-filled form).

Solutions, suspensions and gels normally contain a major amount of water (preferably purified, sterilized water) in addition to the cells. Minor amounts of other ingredients such as pH adjusters (e.g., a base such as NaOH), emulsifiers or dispersing agents, buffering agents, preservatives, wetting agents and jelling agents (e.g., methylcellulose), may also be present. The compositions can be isotonic, i.e., they can have the same osmotic pressure as blood and lacrimal fluid.

The desired isotonicity of the compositions of this invention may be accomplished using sodium chloride, or other pharmaceutically acceptable agents such as dextrose, boric acid, sodium tartrate, propylene glycol or other inorganic or organic solutes. Sodium chloride is preferred particularly for buffers containing sodium ions.

Viscosity of the compositions, if desired, can be maintained at the selected level using a pharmaceutically acceptable thickening agent. Methylcellulose is preferred because it is readily and economically available and is easy to work with. Other suitable thickening agents include, for example, xanthan gum, carboxymethyl cellulose, hydroxypropyl cellulose, carbomer, and the like. The preferred concentration of the thickener will depend upon the agent selected and the desired viscosity. Viscous compositions are normally prepared from solutions by the addition of such thickening agents.

A pharmaceutically acceptable preservative or cell stabilizer can be employed to increase the life of the compositions. Preferably, if preservatives are necessary, it is well within the purview of the skilled artisan to select compositions that will not affect the viability or efficacy of the MAPCs or progeny as described in the present invention.

Those skilled in the art will recognize that the components of the compositions should be selected to be chemically inert. This will present no problem to those skilled in chemical and pharmaceutical principles, or problems can be readily avoided by reference to standard texts or simple experiments (not involving undue experimentation), from this disclosure and the documents cited herein.

Compositions can be administered in dosages and by techniques available to those skilled in the medical and veterinary arts taking into consideration such factors as the age, sex, weight and condition of the particular patient, and the composition form used for administration (e.g., solid vs. liquid). Dosages for humans or other animals can be determined without undue experimentation by the skilled artisan, from this disclosure, the documents cited herein, and the knowledge in the art.

Suitable regimes for initial administration and further doses or for sequential administrations also are variable, may include an initial administration followed by subsequent administrations; but nonetheless, can be ascertained by the skilled artisan, from this disclosure, the documents cited herein, and the knowledge in the art.

### Approaches for Transplantation to Prevent Immune Rejection

In some embodiments, it may be desired that the MAPCs (or differentiated progeny) be treated or otherwise altered prior to transplantation/administration in order to reduce the risk of stimulating host immunological response against the transplanted cells. Any method known in the art to reduce the risk of stimulating host immunological response may be employed. The following provides a few such examples.
1. Universal donor cells: MAPCs can be manipulated to serve as universal donor cells. Although undifferentiated MAPCs do not express MHC-I or -II antigens, some differentiated progeny may express one or both of these antigens. MAPCs can be modified to serve as universal donor cells by eliminating MHC-I or MHC-II antigens, and potentially introducing the MHC-antigens from the prospective recipient so that the cells do not become easy targets for NK-mediated killing, or become susceptible to unlimited viral replication or malignant transformation. Elimination of MHC-antigens can be accomplished by homologous recombination or by introduction of point-mutations in the promoter region or by introduction of a point mutation in the initial exon of the antigen to introduce a stop-codon, such as with chimeroplasts. Transfer of the host MHC-antigen(s) can be achieved by retroviral, lentiviral, adeno associated virus or other viral transduction or by transfection of the target cells with the MHC-antigen cDNAs.
2. Intrauterine transplant to circumvent immune recognition: MAPCs can be used in an intrauterine transplantation setting to correct genetic abnormalities, or to introduce cells that will be tolerated by the host prior to immune system development. This can be a way to make human cells in large quantities in animals or it could be used as a way to correct human embryo genetic defects by transplanting cells that make the correct protein or enzyme.
3. Immune Recognition and Tolerance:

### A. Immune Recognition

Immune responses are controlled by molecular recognition events between receptors on T cells (T cell receptors or TCR) and somatic tissues (class I and II MHC). The TCR/MHC interactions are the antigen specific component of the immune response, enabling recognition between self and foreign antigen. While an immune reaction will only proceed following T cell recognition of a foreign or non-self antigen, additional signaling events are required and function to prevent accidental or autoimmune responses (Buckley, 2003).

Immune recognition can be divided into two phases, sensitization and secondary responses. Sensitization is accomplished by a subset of T cells, T helper cells, interacting with a specialized population of immune cells called dendritic cells. T helper cell recognition of antigen presented by class II MHC complexes on these dendritic or antigen-presenting cells (APC), is critical for initiating both antibody or cytolytic T cell responses. Only a limited number of cells express class II MHC receptors, and these "professional" APC are characterized by not only sensitizing T helper cells with non-self antigen, but also by expressing cytokine cascades that regulate amplification of T cells and control humoral versus cytolytic immune responses. B cells, macrophages, Langherhans cells, and other dendritic cell classes make up the APC compartment. Therefore, only specialized cell types can signal immune responsiveness, including allogeneic reactivity.

The two classes of MHC receptors, class I and II, have structural motifs that cause intracellular association with short peptide segments derived from all genes expressed in a cell. This complex of peptide bound to the MHC receptor on the cell surface is the molecular complex recognized by TCR, and therefore provides the specificity for antigenic recognition by T cells (analogous to a lock-and-key mechanism). Once the immune system has been sensitized and triggered, immune system cells amplify until the antigen is eliminated, and then reside in a resting or memory state to respond if the antigen is re-encountered.

Control of immune reactivity is accomplished in cascades. In addition to the primary recognition of non-self peptides between T helper cells and APC, a second stage is the required stimulation of APC by pathogen associated stimuli - for example, bacterial cell wall components such as LPS, viral particles that cross-link surface Ig on B cells; double-stranded RNA associated with viral infection; or inflammatory cytokines produced by physical wounding and damage to vasculature - all of these provide non-antigen specific confirmation that an immune response is warranted. The nature of these initial signals also triggers the APC to regulate humoral vs cellular responses by stimulating different cytokine cascades.

### B. Tolerance

A second cascade that regulates the immune system is the restriction of the response to self-antigens by eliminating self-reactive T cells. For both B and T cell immunity, this is accomplished by regulating the repertoire of the T helper cell population, as this population determines reactivity in a sensitization reaction. T cells are produced in the bone marrow, and circulate to the thymus for "education" to distinguish between self and non-self antigens. T cells which can recognize self tissue are depleted during ontogeny in the thymus, to ensure that no T cells with T cell receptor complexes (TCR) reactive to self-antigen persist in circulation. This is termed central tolerance, and when broken, results in autoimmune disease.

A second type of tolerance can be induced, known as peripheral tolerance. This is accomplished when T cells that have passed through the thymus encounter non-self antigen, but do not receive secondary or co-stimulatory signals from APC that are required to trigger either helper or cytolytic function. This might occur when an APC has expressed antigen via a class II MHC receptor, but not received accessory signals as a consequence of infection or pathogen threat, and hence the APC does not express the cytokine cascade required for response. T cells partially stimulated in this fashion are rendered anergic or apoptotic. This results in depletion of the T helper population required for humoral or cytolytic responsiveness.

A second form of peripheral tolerance is generated when cytolytic T cells encounter cells expressing non-self antigen in class I MHC complexes on the majority of somatic cells. When the TCR of these T cells engage class I MHC in the absence of co-stimulatory receptor engagement (e.g., CD28/CD86 interaction), the T cells are rendered anergic or apoptotic. There is a panel of secondary co-stimulatory receptor interactions necessary and capable of providing this secondary signal, and therefore the surface phenotype of a cell can strongly predict immune stimulation or anergy.

Many tumor cells have evolved escape pathways from cytolytic recognition by down-regulating class I MHC expression, thus becoming invisible to the T cell arm of the immune system. Many viruses have evolved specific mechanisms for interfering with cell surface expression of MHC receptors in order to escape immune responses. An additional arm of the immune system has evolved to clear tumor cells, or virally infected cells with this property of reduced MHC expression. A population of cells termed natural killer or NK cells are capable of cytolytic activity against class I MHC negative cells. This activity is negatively regulated. NK cells bind target cells through interaction with receptors called Killer Inhibitory Receptors (KIR) and will kill unless turned off by interaction with class I MHC.

### C. Hematopoietic Chimerism and Tolerance Induction

Bone marrow transplant is necessitated in cancer therapy where chemotherapeutic agents and/or radiation therapy results in myeloablation of the host immune system. The patient then reconstitutes immune function from the hematopoietic stem cells present in the bone marrow graft, and therefore has acquired the cellular and molecular components of the immune system from the bone marrow donor. The reconstitution of the donor immune system is accompanied by recapitulation of the self vs. non-self antigenic education seen in ontogeny, whereby the donor immune system is now tolerized to host tissues. A secondary aspect of donor immune system reconstitution is that the host is now capable of accepting an organ or tissue graft from the original donor without rejection.

When less severe myeloablative conditioning is used for bone marrow transplant, the host immune system may not be completely depleted, and with appropriate immunosuppressive management, a chimeric immune system may be reconstituted comprised of both donor and host immune cells. In this setting, the host is tolerized to the cellular and molecular components of both donor and host, and could accept an organ or tissue graft from the bone marrow donor without rejection. The clinical management of host rejection of donor bone marrow, and graft-versus-host response from donor bone marrow is the key to success in this therapeutic approach. The clinical risk of graft-versus-host response is a significant and as yet incompletely resolved risk in standardizing this approach for transplantation. These clinical protocols have received significant attention recently (Waldmann, 2004).

Significant benefit would be achieved through use of a stem cell, capable of reconstituting the immune system, that did not carry risk of graft-versus-host response. The graft-versus-host reaction is due to contaminating T cells inherent in the bone marrow graft. Although purification of hematopoietic stem cells from bone marrow is routine, their successful engraftment in the patient requires accompaniment by accessory T cells. Thus, a critical balance must be achieved between the beneficial engraftment value of T cells and the detrimental effect of graft-versus-host response.

MAPCs and ES cells represent a stem cell population which can be delivered without risk of graft-versus-host reactivity, as they can be expanded free of hematopoietic cell types including T cells. This greatly reduces clinical risk. The transient elimination of NK cell activity during the acute phase of cell delivery increases the frequency of primitive stem cell engraftment and hematopoietic reconstitution to a clinically useful threshold without risk of long term immunosuppression.

As MAPC or ES engraft and contribute to hematopoiesis, the newly formed T cells undergo thymic and peripheral self vs non-self education consistent with host T cells as described above. Co-exposure of newly created naïve T cells of donor and host origin results in reciprocal depletion of reactive cells, hence tolerance to T cells expression allogeneic antigens derived from a MAPC or ES donor can be achieved. A patient can thus be rendered tolerant to the cellular and molecular components of the MAPC or ES donor immune system, and would accept a cell, tissue or organ graft without rejection.

### D. MAPC and Other Stem Cell Types

This above mechanism of tolerance induction is unique to a cell type capable of hematopoietic reconstitution. Although mesenchymal stem cells, also derived from bone marrow, have shown low immunogenicity and can persist in an allogeneic transplant setting, tolerance to donor immune components is not achieved. No other lineage committed stem cell has demonstrated hematopoietic reconstitution potential. This includes neuronal stem cells, fat-derived stem cells, liver stem cells, etc.

The ability to induce tolerance to subsequent graft acceptance using ES cells has been demonstrated by Fandrich (2002). In this setting, non-ablative conditioning accompanied by delivery of a murine ES cell type enabled animals to accept a heart allograft without rejection. Hence, the lineage regenerative properties common to ES cells and MAPC which includes hematopoietic reconstitution can achieve transplant tolerance. MAPCs represent an alternative to clinical use of ES cells for transplant tolerance.

Thus, the administration of MAPC or BS and the differentiation thereof into the various blood cell types can condition or prepare a recipient for secondary organ or tissue transplant with histocompatibility matching to the MAPC or ES cells. For example, a diabetic subject may be treated with cells obtained from, for example, a stem cell bank. Tolerization will follow and then one can provide to the diabetic subject allogeneic islet cells obtained or derived from the same source as the stem cell so that the mature islets are not rejected by the recipient. This process is available for any secondary transplant (e.g., organ, tissue and/or cell transplant) including, but not limited to, heart, liver, lung, kidney and/or pancreas.

### 4. Natural Killer (NK) Cell Function:

Any means, such as an agent, which inhibits NK cell function, including depleting NK cells from a population of cells, may also be administered to prevent immune rejection, increase engraftment or increase immune tolerance. Such an agent includes an anti-NK cell antibody, irradiation or any other method which can inhibit NK cell function. NK function inhibition is further described in PCT Application No. PCT/US2005/015740, filed May 5, 2005 .

Thus, there is also provide herein a method to increase immunologic tolerance in a subject to MAPCs comprising administering a population of the MAPCs and an effective amount of an agent for inhibiting Natural Killer cell function to the subject, so that immunologic tolerance to the MAPCs increases compared to the method without administration of the inhibiting agent.

### 5. Gene Therapy:

MAPCs can be extracted and isolated from the body, grown in culture in the undifferentiated state or induced to differentiate in culture, and genetically altered using a variety of techniques, especially viral transduction. Uptake and expression of genetic material is demonstrable, and expression of foreign DNA is stable throughout development. Retroviral and other vectors for inserting foreign DNA into stem cells are available to those of skill in the art. (Mochizuki, H. et al. 1998; Robbins, P. et al. 1997; Bierhuizen, M. et al. 1997; Douglas, J. et al. 1999; Zhang, G. et al. 1996). Once transduced using a retroviral vector, enhanced green fluorescent protein (eGFP) expression persists in terminally differentiated muscle cells, endothelium and c-Kit positive cells derived from isolated MAPCs, demonstrating that expression of retroviral vectors introduced into MAPC persists throughout differentiation. Terminal differentiation was induced from cultures initiated with about 10 eGFP⁺ cells previously transduced by retroviral vector and sorted a few weeks into the initial MAPC culture period.

### Monitoring of Subject After Administration of MAPCs

Following transplantation, the growth or differentiation of the administered MAPCs or the therapeutic effect of the MAPCs or progeny may be monitored.

Following administration, the immunological tolerance of the subject to the MAPCs or progeny may be tested by various methods known in the art to assess the subject's immunological tolerance to MAPCs. In cases where the subject's tolerance of MAPCs is suboptimal (e.g., the subject's immune system is rejecting the exogenous MAPCs), therapeutic adjunct immunosuppressive treatment, which is known in the art, of the subject may be performed.

### Genetically-Modified MAPCs

MAPCs or differentiated progeny derived therefrom can be genetically altered *ex vivo,* eliminating one of the most significant barriers for gene therapy. For example, a subject's bone marrow aspirate is obtained, and from the aspirate MAPCs are isolated. The MAPCs are then genetically altered to express one or more desired gene products. The MAPCs can then be screened or selected *ex vivo* to identify those cells which have been successfully altered, and these cells can be introduced into the subject or can be differentiated and introduced into the subject, either locally or systemically. Alternately, MAPCs can be differentiated and then the differentiated cells can be genetically altered prior to administration. In either case, the cells provide a stably-transfected source of cells that can express a desired gene product. Especially where the patient's own tissue, such as bone marrow, is the source of the MAPCs, this method provides an immunologically safe method for producing cells for transplant.

### Methods for Genetically Altering MAPCs

Cells isolated by the methods described herein, or their differentiated progeny, can be genetically modified by introducing DNA or RNA into the cell by a variety of methods available to those of skill in the art. These methods are generally grouped into four major categories: (1) viral transfer, including the use of DNA or RNA viral vectors, such as retroviruses, including lentiviruses (Mochizuki, H., et al., 1998; Martin, F., et al. 1999; Robbins, et al. 1997; Salmons, B. and Gunzburg, W.H., 1993; Sutton, R., et al., 1998; Kafri, T., et al., 1999; Dull, T., et al., 1998), Simian virus 40 (SV40), adenovirus (see, for example, Davidson, B.L., et al., 1993; Wagner, E., et al., 1992; Wold, W., Adenovirus Methods and Protocols, Humana Methods in Molecular Medicine (1998), Blackwell Science, Ltd.; Molin, M., et al., 1998; Douglas, J., et al., 1999; Hofmann, C., et al., 1999; Schwarzenberger, P., et al., 1997), alpha virus, including Sindbis virus (U.S. Patent No. 5,843,723; Xiong, C., et al., 1989; Bredenbeek, P.J., et al., 1993; Frolov, I., et al., 1996), herpes virus (Laquerre, S., et al., 1998) and bovine papillomavirus for example; (2) chemical transfer, including calcium phosphate transfection and DEAE dextran transfection methods; (3) membrane fusion transfer, using DNA-loaded membranous vesicles such as liposomes (Loeffler, J. and Behr, J., 1993), red blood cell ghosts and protoplasts, for example; and (4) physical transfer techniques, such as microinjection, microprojectile J. Wolff in "Gene Therapeutics" (1994) at page 195. (see J. Wolff in "Gene Therapeutics" (1994) at page 195; Johnston, S.A., et al., 1993; Williams, R.S., et al., 1991; Yang, N.S., et al., 1990), electroporation, nucleofection or direct "naked" DNA transfer.

Cells can be genetically altered by insertion of pre-selected isolated DNA, by substitution of a segment of the cellular genome with pre-selected isolated DNA, or by deletion of or inactivation of at least a portion of the cellular genome of the cell. Deletion or inactivation of at least a portion of the cellular genome can be accomplished by a variety of means, including but not limited to genetic recombination, by antisense technology (which can include the use of peptide nucleic acids or PNAs), or by ribozyme technology, for example. Insertion of one or more pre-selected DNA sequences can be accomplished by homologous recombination or by viral integration into the host cell genome. Methods of non-homologous recombination are also known, for example, as described in U.S. Patent Nos. 6,623,958, 6,602,686, 6,541,221, 6,524,824, 6,524,818, 6,410,266, 6,361,972.

The desired gene sequence can also be incorporated into the coll, particularly into its nucleus, using a plasmid expression vector and a nuclear localization sequence. Methods for directing polynucleotides to the nucleus have been described in the art. For example, signal peptides can be attached to plasmid DNA, as described by Sebestyen, et al. (1998), to direct the DNA to the nucleus for more efficient expression.

The genetic material can be introduced using promoters that will allow for the gene of interest to be positively or negatively induced using certain chemicals/drugs, to be eliminated following administration of a given drug/chemical, or can be tagged to allow induction by chemicals (including but not limited to the tamoxifen responsive mutated estrogen receptor) in specific cell compartments (including but not limited to the cell membrane).

Successful transfection or transduction of target cells can be demonstrated using genetic markers, in a technique that is known to those of skill in the art. The green fluorescent protein of *Aequorea victoria,* for example, has been shown to be an effective marker for identifying and tracking genetically modified hematopoietic cells (Persons, D., et al., 1998). Alternative selectable markers include the β-Gal gene, the truncated nerve growth factor receptor, drug selectable markers (including but not limited to NEO, MTX, hygromycin).

Any of transfection or transduction technique can also be applied to introduce a transcriptional regulatory sequence into MAPCs or progeny to activate a desired endogenous gene. This can be done by both homologous (e.g., U.S. 5,641,670) or non-homologous (e.g., U.S. 6,602,686) recombination.

### Examples

The following examples are provided in order to demonstrate and further illustrate certain embodiments and aspects of the present invention and are not to be construed as limiting the scope thereof.

### Example 1

### MAPCs Give Rise to HSCs that Reconsititute the Lympho-Hematopoietic System

In 2002, it was published that when murine LacZ⁺MAPCs are transplanted into sublethally irradiated NOD-SCID mice, they contribute to the hematopoietic system; however, with low hematopoietic engraftment levels (2-8% myeloid and B-lymphoid cells, no T-lymphoid cells) (Jiang Y et al. 2002). As demonstrated herein, MAPCs can give rise to up to 95% GFP⁺ blood cells when endogenous NK cells, that are still present in NOD-SCID mice, are eliminated. Additionally, MAPCs isolated and cultured under low O₂ (5%) conditions, results in MAPCs that have higher mRNA and protein levels of the ES-transcription factor, Oct 4, and greater differentiation potential. Thus, the engraftment of low O₂ MAPCs in NOD-SCID mice irradiated with 275cGy and treated with anti-asialo GM1 antibody on d1, d11 and d22, to decrease NK activity, was investigated.

Murine MAPC cell lines were established from eGFP transgenic C57B1/6 Thy1.1 mice bone marrow cells as described in Jiang, Y. et al. 2002. MAPCs were cultured in 60% DMEM-LG (Gibco BRL), 40% MCDB-201 with 1x SITE, 0.2x LA-BSA, 0.2 g/l BSA, 0.1 mM ascorbic acid 2-phosphate, 0.1 mM betamercaptoethanol (Sigma), 100 U penicillin, 1000 U streptomycin (Gibco), 1000 U/ml LIF (Chemicon), 10 ng/ml mEGF (Sigma), 10 ng/ml hPDGF-BB (R&D systems), 2% fetal calf serum (FCS) (Hyclone Laboratories) on a human 10 ng/cm² fibronectin (Sigma)-coated dish (Nunc) at about 5% CO₂ and about 5% O₂. Plating cell density was about 100 cells/cm² and cells were split every two days.

About 0.3-1 x 10⁶ 5% O₂ cultured eGFP C57B1/6 MAPCs were transplanted via tail vein injection in 6-8 week old NOD-SCID mice (n=11) following irradiation at 275cGy. Intraperitoneal injection of anti-asialo-GM1 antibody (Wako) (20µl of the stock solution diluted in 380µl of PBSlx) was given on day -1, +10 and +20 to decrease NK activity (Table 2).

**Table 2: Lymphohematopoietic Engraftment from mMAPC**

| | | | Engraftment level (%) | | |
|---|---|---|---|---|---|
| Animal | #cells injected | Time (weeks) | Marrow | Blood | Spleen |
| 1 | 1x10⁶ | 12 | 0 | 0 | 0 |
| 2 | 1x10⁶ | 12 | 0 | 0 | 0 |
| 3 | 1x10⁶ | 12 | 0 | 0 | 0 |
| 4 | 1x10⁶ | 5 | 15.2 | n.d. | 56.1 |
| 5 | 0.3x10⁶ | 7 | 2.5 | 37.6 | 16 |
| 6 | 1x10⁶ | 13 | 68 | 92 | 84 |
| 7 | 1x10⁶ | 6* | n.d. | 11 | n.d. |
| 8 | 1x10⁶ | 6* | n.d. | 15 | n.d. |
| 9 | 1x10⁶ | 6* | n.d. | 18 | n.d. |
| 10 | 1x10⁶ | 20 | >90 | >90 | >90 |
| 11 | 1x10⁶ | 20 | >90 | >90 | >90 |

| | | | | | |
|---|---|---|---|---|---|
| * Animals still alive | | | | | |

MAPCs transplanted in animals 1-3 had low levels of Oct 4 (<0.1% of mESCs), whereas all other MAPCs had Oct 4 mRNA levels >30% of mESCs.

Lymphohematopoietic reconstitution was assessed in peripheral blood (PB) at periodic intervals after transplantation (4-10 weeks). Animals 4 and 5 were sacrificed at 5 and 7 weeks following grafting because they appeared ill. Animals 1-2, 6 and 10-11 were sacrificed between 12 and 20 weeks. Animals 7-9 are still alive and only PB has been examined for GFP positive cells. In all animals that were sacrificed, blood, BM, and spleen were evaluated for the presence of eGFP lymphohematopoietic cells. In addition, tissues were harvested to determine contribution of MAPC-derived cells to non-lymphohematopoietic lineages.

As described in Table 2, 3/11 animals (animals 1-3 with low levels of Oct 4) did not have signs of MAPC-derived lymphohematopoiesis. In the other 8 animals, there was evidence of increasing levels of eGFP positive lymphohematopoiesis (data not shown), reaching ~95% by week 20. When animals were evaluated before 10 weeks, levels of engraftment were low, suggesting that conversion from MAPCs to HSCs (which then will give rise to more mature hematopoietic cells) may be protracted compared with grafting of HSC. Consistent with this possibility is that if MAPCs are transplanted in lethally irradiated (9Gy) FANC-C mice without co-injection of compromised BM cells (BM minus stem cells; supports early heamtopoietic recovery, but not long term engraftment), recipient animals die aplastic after 14-21 days (as discussed herein below). However, when compromised BM is also administered, animals survive, and eventually develop GFP⁺ MAPC-derived lymphohematopoiesis. Based on these observations, this dose of MAPCs may not be able to rescue animals from lethal irradiation in an acute setting, but may give rise to LTR (long-term repopulating)-HSCs. Such LTR-HSCs may express cKit and Sca1 and may give rise to functional lymphohematopoiesis in secondary recipients. Additionally, MAPC-derived HSCs may generate functional T- and B-cells, which may populate secondary lymphoid organs including thymus, spleen and lymph nodes, and be capable of mounting an immune response to Keyhole limpet hemocyanin (KLH).

Analysis of lymphohematopoietic tissues in mouse #6 demonstrated multi-lineage engraftment (Figure 2A). First, normal size of spleen, thymus and lymph nodes were seen in MAPC-transplanted NOD-SCID mice. In addition, Figure 2 shows that these organs are populated by eGFP⁺ cells.

FACS analysis was done on single cell suspensions of PB, BM, spleen and thymus. Results are shown in Figure 2B-C for mouse #6. There are clearly distinguishable eGFP⁺CD45.2⁺ (BD Biosciences Pharmingen) cells in the BM, PB, spleen and thymus (up to 70% GFP/CD45.2 cells were present in PB, BM and spleen at 13 weeks post-transfer). In the PB, eGFP⁺ cells co-expressed B220, CD19, CD3, CD4, CD8, NK1.1, Macl, and GR1 (BD Biosciences Pharmingen). Analysis of the BM revealed 68% eGFP⁺ cells, co-expressing mature hematopoietic markers, including CD34PE, CD45.2APC, Thy 1.1PE, Sca-1PE, c-Kit APC (BD Biosciences Pharmingen). The frequency of T cells is low. This is not unexpected because one would expect a progressive increase in T cell production over time in view of the slow immune reconstitution of MAPCs in rudimentary mouse thymus and thymic recovery observed prior to peripheralization of T cells. In addition ~1% of the GFP⁺ cells co-expressed Scal⁺/cKit or Thy1⁺/cKit (BD Biosciences Pharmingen). This suggests generation of HSC from MAPC, consistent with the finding that GFP⁺CFU-Mix, BFU-E and granulocyte-monocyte-colony-forming units (CFU-GM) were present.

FACS analysis of cells from spleen, BM and peripheral blood revealed the presence of multilineage engraftment with differentiation to myeloid (Mac-1/Gr-1), B-(B220/CD19/IgM) and T-(CD4/CD8/TCR*αβ*) lymphoid cells. For example, in the spleen (Figure 2C), differentiation to cells with B-lymphoid (CD19PE, B220APC, IgMAPC (BD Biosciences Pharmingen) and T-lymphoid (CD4APC and CD8APC (BD Biosciences Pharmingen) cell phenotype were detected. eGFP sorted splenic T cells were capable of reacting to Balb/C derived cells in a mixed lymphocyte reaction culture and to stimulation by anti-CD3 ⁺ anti-CD28 mAbs (antibodies provided by Dr. Carl June; Brice et al. 1998). eGFP⁺ cells in the thymus that expressed CD4, CD8 and TCR*β* (BD Biosciences Pharmingen) were also detected. Gross examination of the gut also identified eGFP-containing Peyer's patches.

### Example 2

### MAPCs can differentiate into HSC/HPC in vitro

Hematopoietic cells can be generated from murine ESCs, using either feeder cells such as OP935, or by allowing the ES cells to form embroid bodies (EBs) (Choi K et al. 1998), and then subsequently inducing hematopoietic cells using cytokines known to act at the mesoderm-hemangioblast interphase, and subsequently later-acting hematopoietic cytokines (Faloon P et al. 2000; Schuh AC et al. 1999). ESCs sequentially express hemangioblast markers, including Flkl, SCL, and LMO2 and subsequently hematopoietic markers. A sequential activation of primitive and then definitive hematopoiesis is seen. During development, definitive HSCs arising in the AGM region are c-Kit and AA4.1 positive. Of note, it is thought that the panhematopoietic marker, CD45, is expressed following acquisition of CD41 during development, and that expression of CD41, but not CD45, indicates commitment to an LTR-HSC fate (Mikkola HK et al. 2003; Bertrand JY et al. 2005).

When hematopoietic cells generated *in vitro* from mESCs are grafted in post natal animals, no or very minimal engraftment is seen (Potocnik AJ et al. 1997). Kyba et al. (2002; 2003) have recently disclosed that when HoxB4 or Stat5A is expressed short term within mESC derived hematopoietic cells, engraftment *in vivo* is possible, even though lymphoid reconstitution is not very robust. Similar *in vitro* differentiation from hESCs has been achieved (Kaufman DS et al. 2001; Tian X et al. 2004; Vodyanik MA et al. 2005; Wang L et al. 2004; Cerdan C et al. 2004). Several studies have suggested that hESC derived hematopoietic cells engraft in irradiated immunodeficient animals, even though levels of engraftment are low.

To support the steps that govern specification and commitment of cells to hemangioblasts and HSCs *in vitro,* a number of stromal cell lines have been created from hemogenic microenvironments including adult BM, fetal liver and AGM, of which some support primitive progenitors. Two of these cell lines, EL08-1D2 and UG26-1B6 (Oostendorp RA et al. 2002; Buckley S et al. 2004) support both human and mouse hematopoietic progenitor cells (HPCs) in long-term culture (LTC), and both feeders support murine repopulating HSCs (Kusadasi N et al. 2002; Oostendorp RA et al. 2002; Harvey K et al. 2004; Oostendorp RA et al. 2002), and as described herein below, the EL08-1D2 cell line can specify MAPCs *in vitro* to a lymphohematopoietic fate. In addition, a number of cytokines and factors have been identified that are responsible for the early hematopoietic specification and commitment. These include factors that act at the mesoderm-HSC interphase as well as factors with known activity at the HSC level, such as members of the TGF*β*/BMP family (Leung AYH et al. 2004) or Wnt (Reya T. 2003) family, IHH (Dyer MA et al. 2001), VEGF (Choi K 1998) or bFGF (Faloon P et al. 2000) as well as early acting hematopoietic cytokines, including SCF, Flt3L and Tpo.

A. mMAPC Differentiate into lymphohematopoietic Cells Following Co-Culture with EL08-1D2 cells. Murine MAPC cell lines were established from eGFP transgenic mice mice bone marrow cells as described in Jiang, Y. et al. (2002) at about 5% O₂. 10⁴ eGFP⁺ mMAPCs were cultured for 14 days in contact with the E11.5 murine embryonic liver feeder (EFL) EL08-1D2 cells (grown to confluence and irradiated with 2500 cGy of Cesium; obtained from Dr. E. Dzierzak, Rotterdam; Oostendorp RA et al. 2000) in 10% FCS containing medium (Myelocult M5300 (Stem Cell Technologies)) with 20 ng/ml mSCF (R&D Systems), 10 ng/ml mTpo (R&D Systems), 10 ng/ml mIL3(R&D Systems), 10 ng/ml mIL6 (R&D Systems), followed by 14-16 days in CFC cultures using Methocult medium (MethoCult™ methylcellulose-based medium (Stem Cell Technologies)) containing 20 ng/ml mSCF (R&D Systems), 10 ng/ml mIL-3 (R&D Systems), 10 ng/ml mIL6 (R&D Systems), and 3 U/ml hEpo. Cells were cultured at 37°C in the presence of 1X *β*-mercaptoethanol (Gibco) with 5% CO₂.

Undifferentiated MAPC and d14 MAPC were evaluated by Q-RT-PCR for hematopoietic, endothelial and endodermal markers; d14-16 Methocult cultures were scored for presence of CFC. Q-RT-PCR analysis of undifferentiated MAPCs did not detect LMO2, SCL, GATA1 or PU.1 mRNA and very low levels of GATA2 mRNA, and FACS analysis demonstrates that undifferentiated mMAPCs are cKitPos, but ScalNeg, CD34Neg, CD41Neg, CD45Neg, ThylNeg and LinNeg (antibodies were purchased from BD Pharmingen). Lineage Cocktail contains Gr-1, Mac-1, Terr-119, CD4, CD8, and B220 biotinylated.

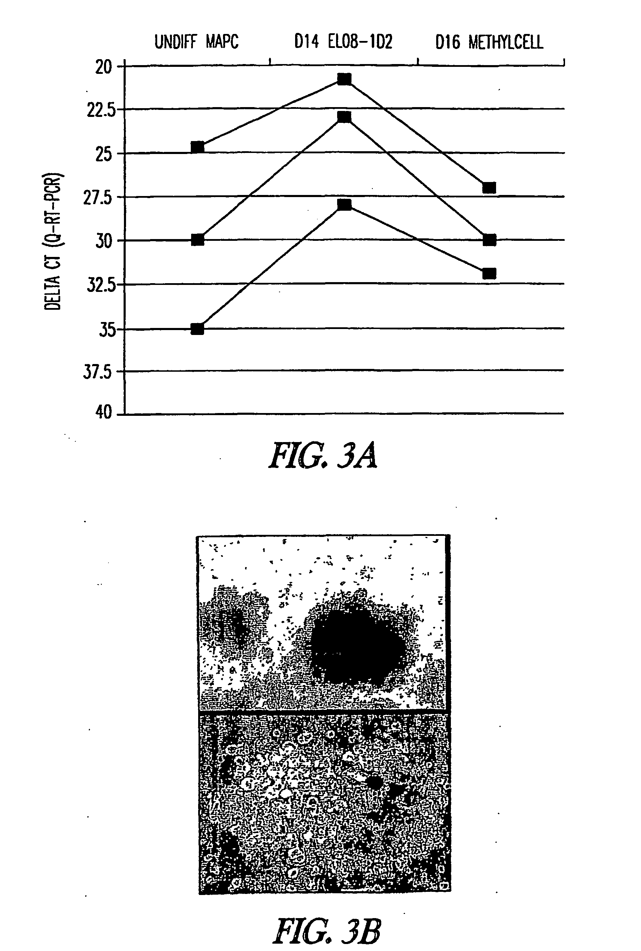

Transcripts for hematopoietic transcription factors (GATA2, GATA1, LMO2, SCL, PU.1) were expressed by day 14, and increased further by d28 (Figure 3A). However, markers of more mature hematopoietic cells (CD45, MPO, Hbγ and Hb*β*) were not expressed on d14, but were highly expressed by d28. Of note, Hbγ and Hb*β* were identified, suggesting definitive hematopoiesis, not embryonic hematopoiesis. Transcripts for endothelial cells (Flk1, VE-cadherin, vWF) were also found to be expressed significantly higher by d14; however, levels decreased by d28, suggesting that the first stage of differentiation may occur via a hemangioblast intermediate, similar as described for mESCs (Choi K. 1998; Choi K et al. 1998). CFU-Mix colonies were also generated (Figure 3B).

### B. Human (h)MAPC also Differentiate into Lymphohematopoietic Cells.

Human MAPC cell lines were established and cultured as described herein. eGFP transduced MAPCs, that are GlyA, CD45 and CD34 negative (n=20), were cocultured with the mouse yolk sac mesodermal cell line, YSM5, as suspension cell aggregates for 6 days in serum free medium supplemented with 10 ng/mL bFGF and VEGF. After 6 days, only eGFP⁺ cells (i.e., MAPC progeny) remained and YSM5 cells had died.

Remaining cells were transferred to methylcellulose cultures containing 10% fetal calf serum (FCS) supplemented with 10 ng/mL bone morphogenic protein (BMP) 4, VEGF, bFGF, stem cell factor (SCF), Flt3L, hyper IL6, thrombopoietin (TPO) and erythropoietin (EPO) for 2 weeks. In these cultures, both adherent eGFP⁺ cells and small, round non-adherent cells, which formed many colonies attached to the adherent cells were detected. The non-adherent and adherent fractions were collected separately and cultured in 10% FCS containing medium with 10 ng/mL VEGF and bFGF for 7 days. Adherent cells stained positive for vWF, formed vascular tubes when plated on ECM, and were able to uptake a-LDL, indicating their endothelial nature. 5-50% of the non-adherent cells stained positive for human specific GlyA and HLA-class I by flow cytometry. Gly-A⁺/HLA-class-I⁺ cells were selected by FACS. On Wright-Giemsa, these cells exhibited the characteristic morphology and staining pattern of primitive erythroblasts. Cells were benzidine⁺ and human Hb⁺ by immunoperoxidase. By RT-PCR these cells expressed human specific Hb-e, but not Hb-a.

When replated in methylcellulose assay with 20% FCS and EPO, small erythroid colonies were seen after 10 days, and 100% of these colonies stained positive for human specific GlyA and Hb. As selection of MAPCs depends on the depletion of CD45⁺ and GlyA⁺ cells from BM, and cultured MAPCs are CD45⁻ and GlyA⁻ at all times using both FACS and cDNA array analysis, contamination of MAPCs with hematopoietic cells is not likely.

### Example 3

### MAPC Contribution to Blood in FANCC-/- Mice: Gene Repair of FANCC-/- MAPCs

Fanconi anemia (FA) is a severe bone marrow (BM) failure syndrome transmitted through autosomal recessive inheritance. There are at least eleven FA genes (A, B, C, D1 (BRCA2), D2, E, F, G, I, J and L). These eleven account for almost all of the cases of Fanconi anemia. Mutations in FA-A, FA-C and FA-G are the most common and account for approximately 85% of the FA patients worldwide. FA-D1, FA-D2, FA-E, FA-F and FA-L account for 10%. FA-B, FA-I and FA-J represent less than 5% of FA patients. Most of the Fanconi anemia genes have been cloned.

FA occurs equally in males and females. It is found in all ethnic groups. The clinical manifestation of FA is defined by a progressive bone marrow failure and in the majority of cases, a multitude of congenital malformations (Liu JM. 2000). In addition, FA patients are at an increased risk of developing myelodysplasia, acute myelogenous leukemia (AML) and solid tumors later in life (Alter BP. 1992). For example, FA patients are also likely to develop head and neck, gynecological and gastrointestinal squamous cell carcinomas.

The primary mode of long-term curative treatment of the hematological manifestation of the disease is bone marrow (BM) or peripheral blood stem cell (PBSC) transplantation using an allogeneic donor (Gluckman E. 1993; Kohli-Kumar M. et al. 1994; Guardiola P et al. 2000). However, these efforts only address the hematological defects and not the accompanying epithelial defects. Additionally, since the transplant involves allogeneic donor cells, there is a risk of rejection of the donor cells by the recipient. Efforts to use autologous bone marrow cells that have undergone *ex vivo* gene correction has remained largely unsuccessful due to various problems, such as inefficient gene transfer and the poor ability of bone marrow stem cells to be cultured *in vitro.*

Multipotent adult progenitor cells (MAPCs) are a population of stem cells within the adult bone marrow that not only differentiate into lymphohematopoietic cells *in vivo,* but also engraft in, for example, hepatic, gastrointestinal, lung epithelium and endothelium. Additionally, MAPCs can be expanded/cultured for extended periods of time without loss of differentiation potential and they are amendable to genetic manipulation (e.g., for use in gene therapy). Thus, MAPCs are an ideal source for FA treatment.

### Materials and Methods

Isolation and enrichment of MAPC's from bone marrow of FANCC+/+ mouse. FANCC -/- mice that carry a disrupted exon 9 of the FANCC murine homolog gene and its syngeneic normal control FANCC+/+ mice were provided by Dr Markus Grompe. Bone marrow was collected from the femurs of the mice. MAPCs were cultured according to Jiang et al. (2002) with slight modification. Briefly, bone marrow mononuclear cells were obtained by the Ficoll-Plaque density gradient centrifugation (Sigma Chemicals Co., St Louis, MO). The mononuclear cells were plated and depleted using micromagnetic beads (Miltenyi Biotec, Sunnyvale, CA). 5,000 CD45⁻GlyA⁻ cells were plated in 1 ml MAPC expansion medium that consists of DMEM-LG (58%; Gibco-BRL, Grand Island, NY), MCDB-201 (40%; Sigma Chemical Co, St Louis, MO), 2% FCS (Hyclone Laboratories, Logan, UT) supplemented with 1X insulin-transferrin-selenium (ITS), 1X linoleic acid-bovine serum albumin (LA-BSA), 10⁻⁸ M dexamethasone, 10⁻⁴ M ascorbic acid 2-phosphate (AA), 100U penicillin and 1,000U streptomycin in wells of 96 well plates that were coated with 10 mg/ml fibronectin (FN). Culture media was supplemented with 10 ng/ml EGF and 10 ng/ml PDGF-BB (R&D Systems, Minneapolis, MN) and 10 ng/ml Leukemia Inhibitory factor. Once 50% confluent, cells were detached with trypsin/EDTA (Sigma) and replated at a 1/2 dilution in larger culture vessels to keep cell concentrations between 0.8 and 2 X 10³ cells/ cm². The cells were also cultured in the presence of 1X *β*-mercaptoethanol (Gibco) and at 5% O₂.

Analysis of MAPCs. Morphology: Established FANCC+/+ MAPC are shown as small spindle-shaped cells characteristic of MAPC (Jiang et al. 2002). The phenotype of isolated cells were analyzed using FACS based on the expression of surface markers and by Q-RT-PCR for the presence of stem cell markers such as Oct 4 and nanog (Jiang et al, 2002).

Differentiation of MAPCs. Isolated MAPCs were evaluated for multilineage potential by testing their ability to differentiate to endothelium, neuroectodermal and hepatocyte-like cells as previously described (Jiang et al. 2002).

Transplantation of Normal Syngeneic (FANCC+/+) MAPCs into FANCC-/- mouse. Lentiviral marking of FANCC+/+ MAPC: Prior to transplantation, MAPCs were transduced with lenti-GFP. However, transgenic GFP MAPCs can also be used. GFP⁺ MAPCs were infused by tail vein injection into FANCC-/- mice according to Jiang et al. 2002. Briefly, 1 X 10⁶ undifferentiated MAPC, along with 200,000 compromised bone marrow cells (Sca-1 depleted cells), were injected via tail vein into about 7.5 Gy to about 9.0 Gy irradiated 6-9 week old FANCC-/- mice. FANCC-/- mice transplanted with compromised bone marrow cells alone served as controls. FACS analysis of peripheral blood was routinely carried out following 4-6 weeks of transplantation. Eight to ten weeks after transplantation, the animals were sacrificed and contribution of MAPC to hematopoietic organs, such as peripheral blood and bone marrow, were analyzed by FACS. For example, peripheral blood and bone marrow of the transplanted mice was isolated at 8-10 weeks post transplantation. The samples were depleted of red blood cells and labeled with the blood marker CD45 and analyzed by FACS. Cells positive for GFP and CD45 represent blood cells derived from the GFP⁺ MAPC.

Contribution of GFP⁺ MAPC to non-hematopoietic tissue was assessed by immunohistochemistry for GFP and by Q-PCR analysis of the genomic DNA for GFP.

### Results

Lentiviral green fluorescent protein (GFP) transduced, normal MAPCs injected into sublethally irradiated FANCC-/- mice (a Fanconi anemia group C knockout mouse model) contributed to the host hematopoietic system. At 8-10 weeks after transplantation, 2-5% of CD45⁺ cells in the bone marrow of these mice were donor derived GFP⁺ cells. GFP positive cells were also detected in peripheral blood (PB) as well as various tissues harvested from the transplanted animals such as liver, lung and muscle.

As demonstrated herein, the use of high Oct 4 expressing MAPC into NOD-SCID mice depleted of their NK cells give rise to at least 80% hematopoietic repopulation. Similar conditions were carried out in the FANCC-/- mice to achieve higher levels of engraftment. To further selectively repopulate the engrafted cells in the FANCC-/- bone marrow, animals were treated with cyclophosphamide (cyclophosphamide is in a class of drugs known as alkylating agents; it slows or stops the growth of cancer cells) at a dose toxic to host bone marrow cells (e.g., FANCC-/- cells), but not to the normal MAPC derived hematopoietic cells (about 40 mg/kg of body weight).

Thus, host MAPCs from a subject suffering from FA can be isolated, cultured and subjected to *ex vivo* correction of the genetic defect. The cells can then be used as an autologous source of cells for FA treatment of the subject (e.g., long term reconstitution of *ex vivo* gene corrected FA MAPCs in FA subjects).

### Example 4

### Use of Autologous MAPCs to Treat Chronic Myelogenous Leukemia (CML)

Chronic Myelogenous Leukemia (CML) is a clonal myeloproliferative disorder of the HSC, characterized by the Philadelphia chromosome (Ph) and the BCR/ABL fusion gene (Rowley J 1990). HSC transplantation and IFN-α therapy have been the mainstay for therapy of CML for the last 2 decades. More recently, the specific p210BCR/ABL TK inhibitor, Imitinab (Gleevec™; Novartis Pharmaceuticals Corporation (East Hanover, NJ)), has become first line therapy for patients with CML. However, a fraction of patients treated with Imatinib do not achieve cytogenetic remission (CR), and there is evidence that patients that achieve a molecular remission may relapse (Kantarjian HM et al. 2003; Gambacorti-Passerini CB et al. 2003). For these patients, other therapeutic approaches need to be evaluated.

One possibility is to use autologous HSCs harvested at the time of cytogenetic remission. However, there is evidence that even when patients are in CR following Imatinib treatment, malignant HSCs persist (Bhatia R et al. 2003). It has been demonstrated that autografts in CML may result in CRs; however, few of them are long-term (Barnett MJ et al. 1994; Verfaillie C et al. 1998; Carella AM et al. 1997). As identical twin transplants result in at least 5-fold higher long-term remissions (Thomas E et al. 1986), these observations are consistent with the fact that most of the grafts that were reinfused were contaminated with malignant cells (Deisseroth AB et al. 1994).

However, MAPCs generated from the BM of patients with CML, may not harbor the Ph chromosome or the BCR/ABL gene arrangement - as non-hematopoietic cells in BM cultures (stromal cells) from patients with CML are Ph⁻ (Bhatia R et al. 1995). MAPCs may therefore constitute a population of stem cells that can be used to autograft patients with CML.

Thus, hMAPCs in CML patients may be Ph⁻-BCR/ABL⁻ and may give rise to benign long-term repopulating (LTR) HSCs and mature hematopoietic progeny *in vivo.* BM will be obtained from 10 newly diagnosed CML patients (e.g., patients that have not yet been exposed to chemotherapy or irradiation) and MAPCs will be isolated. MAPC populations will be tested for the presence of the Ph chromosome and BCR/ABL gene using standard methods. Cytogenetic stability will be followed over time, as will telomerase activity and telomere lengths. Cells will be tested for their ability to differentiate into mesenchymal cell types, endothelial cells, hepatocyte- and neuroectoderm-like cells, as has been described (Reyes M et al. 2001; Schwartz RE et al. 2002; Reyes M et al. 2002). Once MAPC lines have been established, their ability to generate lymphohematopoietic cells *in vitro* and *in vivo* will be tested, as described herein for non-leukemia (NL) BM derived MAPCs.

As few as 10 cKit⁺Lin⁻Sca1⁺ (KLS) murine cells can give rise to robust hematopoiesis post-grafting, yielding myeloid, B-lymphoid and T-lymphoid cells (Spangrude G et al. 1988). When huUCB CD34⁺Lin⁻CD38⁻ cells are grafted in NOD-SCID mice, a minimum of 500 cells is required to detect >1% human hematopoietic cells, indicating that engraftment across xenogeneic barriers is much less effective. In addition, human hematopoiesis in NOD-SCID mice yields a preponderance of B-cells and some myeloid cells (Hogan CJ et al. 1997; Bhatia M et al. 1998), but no T-cells. However, when human CD34⁺ cells are grafted in BNX (Dao MA and Nolta JA. 1998), NOD-SCID-IL2γcR^{-/-} (Yahata T et al. 2002) or IL2γc/Rag2^{-/-} (Traggiai E et al. 2004) mice, one can also detect T-lymphocytes, that generate a functional human immune response. Like for huCD34⁺ cells, engraftment levels of huMAPC are lower than with muMAPCs, thus the cell dose to be used will be adjusted to include about 10⁷ cells/mouse, in animals that have received near ablative doses of irradiation (700cGy).

Human MAPCs (about 10⁵-10⁷ cells/animal) will be transplanted into 6-8 week old irradiated IL2Rγc/Rag2^{-/-} mice. PB will be evaluated at weeks 4-16 for signs of human lymphohematopoiesis. At 16 weeks, animals will be sacrificed, PB, BM, spleen, thymus and lymph nodes evaluated for huCD45⁺ cells by FACS. As an additional marker to track the cells *in vivo,* huMAPCs transduced with an eGFP containing lentiviral vector will be used. To assess generation of HSCs *in vivo,* secondary transplants with whole bone marrow will be performed, and if successful subsequently with selected huCD34⁺Lin⁻ cells.

Lymphohematopoietic specification and commitment from hMAPCs *in vitro* will be carried out using methods to commit mMAPCs (discussed above) to the lymphohematopoietic lineage, as methods to differentiate hESCs to hematopoietic cells appear in general similar to those used to commit mESCs to hematopoietic cells (Nakano T et al. 1994; Vodyanik MA et al. 2005). As discussed herein above, hMAPC can be committed to the lymphohematopoietic lineage by co-culture with OP9 feeder cells in the presence of VEGF, BMP4, bFGF and hematopoietic cytokines.

The hMAPC-progeny obtained above will be transplanted into irradiated IL2Rγc/Rag2^{-/-} mice. In initial studies, bulk-culture MAPC-progeny will be transplanted. If these cells engraft, then the phenotype of the engrafting cell will be identified by selecting cells based on the expression of CD34, CD41a, CD43, CD45, Thy1, cKit, and/or CD133 - first testing their ability to generate colony forming cells (CFCs) *in vitro* and subsequent transplantation *in vivo.* To demonstrate that long-term repopulating cells were generated, a number of secondary transplants will be performed.

Thus, a non-malignant, non-embryonic stem cell is available for autografting of hematopoietic malignancies, including CML, and potentially other lymphohematopoietic disorders, such as aplastic anemia or inherited genetic disorders of the lymphohematopoietic system.

### Bibliography

Abe, A., et al., J. Virol. 1998;72: 6159-6163.
Akimenko, M.A., J Neurosci 1994;14:3475-86.
Alter BP, Cancer Genet Cytogenet. 1992;58:206-8; discussion 209.
Alvarez-Dolado M., et al. Nature. 2003;425:968-973.
Aranguren XL, et al., The arterial and venous potential of human MAPC and AC133 derived endothelial cells is modulated by activation of notch and patched ligands. Keystone Symposium on stem cells. 2005.
Babcook, et al., Proc. Natl. Acad. Sci. (USA). 1996;93: 7843-7848.
Barker JN and Wagner JE. Nature Reviews Cancer. 2003;3:526-532.
Barker JN et al., Blood. 2003;102:1915-1919.
Barker JN et al., Blood. 2004;ePub.
Barnett MJ et al., Blood. 1994;84:724-732.
Basch, et. al., J. Immunol. Methods. 1983;56:269.
Batinic, D., et al., Bone Marrow Transplant. 1990;6(2):103-7.
Ben-Shushan E et al., Mol Cell Biol. 1998;18:1866-1878.
Bertrand JY et al., Proc Natl Acad Sci U S A. 2005;102:134-139.
Bhardwaj G et al. Nat Immunol. 2001;2:172-180.
Bhatia M et al., Nat Med. 1998;4:1038-1045.
Bhatia R et al., Blood. 1995;85:3636-3645.
Bhatia R et al., J Clin Invest. 1994;94:384-391.
Bhatia R et la., Blood. 2003;101:4701-4707.
Bierhuizen, M. et al., Blood. 1997;90(9):3304-3315.
Bird, et al., Science. 1988;242:423-426.
Bittira B, et al. Eur J Cardiothorac Surg. 2003;24:393-398.
Bjorklund LMea. Proc Natl Acad Sci U S A. 2002;99:2344-2349.
Borue X, et al. Am J Pathol. 2004;165:1767-1772.
Bredenbeek, P.J., et al. J. Virol. 1993;67:6439-6446.
Brice, G.T., et al., J. Acquir Immune Defic Syndr Hum Retrovirol. 1998;19:210-220.
Buckley S et al., Stem Cells. 2004.
Cai Z.H., et al., Artif Organs. 1988;12(5):388-93.
Camargo FD et al., J Clin Invest. 2004;113:1266-1270.
Carella AM et al., Blood Rev. 1997;11:154-159.
Cerdan C et al., Blood. 2004;103:2504-2512.
Chambers I et al., Cell. 2003;113:643-655.
Chang, P., et al., Trends in Biotech. 1999;17:78-83.
Chang, T.M., Artif Organs. 1992;16(1):71-4:
Choi K et al., Biochem Cell Biol. 1998;76:947-956.
Choi K et al., Development. 1998;125:725-732.
Clackson et al. Nature. 1991;352:624-628.
Clarke, Science. 2000;288:1660-3.
Clavel C et al., Isolation and Characterization of Multipotent Adult Progenitor Cells from Cynomologus Monkey. Keystone Synposium on stem cells. 2005.
Clothia et al., J. Mol. Biol. 11985;186:651-66, 1985.
Coligan, et al., Current Protocols in Immunology, (1991 and 1992).
Dao MA and Nolta JA. Int J Mol Med. 1998;1:257-264.
Davidson, B.L., et al., Nature Genetics. 1993;3:219-223.
Deisseroth AB et al., Blood. 1994;83:3068-3076.
Douglas, J., et al. Nature Biotech. 1999;17:470-475.
Douglas, J. et al., Hum. Gene Ther. 1999 ;10(6):935-945.
Drukker M, et al. Proc Natl Acad Sci USA. 2002;99:9864-9869.
Dull, T., et al., J. Virol. 1998;72:8463-8471.
Dyer MA et al., Development. 2001;128:1717-1730.
Eckfeldt CE et al., PLoS Biology. 2004.
Faloon P et al., Development. 2000;127:1931-1941.
Ferrari, Science. 1998;279:528-30.
Foerst-Potts, L. Dev Dyn 1997;209:70-84.
Frolov, I., et al. (Proc. Natl. Acad. Sci. USA. 1996;93:11371-11377.
Gambacorti-Passerini CB et al., Lancet Oncol. 2003;4:75-85.
Gluckman E. Stem Cells 1993;11 Suppl 2:180-3.
Grant MB et al., Nat Med. 2002;8:607-612.
Guardiola P, et al., Blood. 2000;95:422-9.
Guinan EC et al., J Pediatr 1994;124:144-50.
Gunsilius E et al., Lancet. 2000;355:1688-1691.
Gupta P et al., Blood. 1996;87:3229.
Gupta P et al., Blood. 1998;92:4641-4651.
Gussoni, Nature. 1999;401:390-4.
Harraz M et al., Stem Cells. 2001;19:304-312.
Harris RG et al., Science. 2004;305:90-93.
Harvey K and Dzierzak E. Stem Cells. 2004;22:253-258.
Hematti P et al., PLOS Biology. 2004;2:e243.
Hemmati-Brivanlou A et al., Dev Genet. 1995;17:78-89.
Hofmann, C., et al. J. Virol. 1999;73:6930-6936.
Hogan CJ et al., Blood. 1997;90:85-96.
Hollinger et al., Proc. Natl. Acad Sci. USA. 1993;906444-6448 (1993).
Holmes, et al., J. Immunol. 1997;158:2192-2201.
Holyoake TL et al., Exp Hematol. 1999;27:1418-1427.
Howe CWS, Radde-Stepanick T. Hematopoietic Cell Donor Registries. In: Thomas ED, Blume, Karl G. and Forman, Stephan J., ed. Hematopoietic Cell Transplantation. Vol. 2. Malden, MA: Blackwell Sciences; 1999:503-512.
Hurley RW et al., J Clin Invest. 1995;96:511-521.
Jackson, PNAS USA. 1999;96:14482-6.
Jahagirdar, B.N., et al. Exp Hematol. 2001;29(5):543-56.
Jiang Y et al., Blood. 2000;95:846-854.
Jiang Y et al., Proc Natl Acad Sci USA. 2000;97:10538-10543.
Jiang Y HD et al., Proc Natl Acad Sci U S A. 2003;100 Suppl 1:11854-11860.
Jiang Y, et al., Exp Hematol. 2002b;30:896-904.
Jiang Y, et al., Nature. 2002a;418:41-49.
Jiang Y, et al., Proc Natl Acad Sci U S A. 2003;100 Suppl 1:11854-11860.
Johnston, S.A., et al., Genet. Eng. (NY) 1993;15: 225-236.
Jones et al., Nature. 1986;321:522-525.
Kafri, T., et al., J. Virol. 1999;73:576-584.
Kannagi, R. EMBO J 1983;2:2355-61.
Kantarjian HM et al., Blood. 2003;101::97-100.
Kaufman DS et al., Proc Natl Acad Sci USA. 2001;98:10716-10721.
Kawada H, et al. Blood. 2004;104:3581-3587.
Kemahli S et al., Br J Haematol 1994;87:871-2.
Kogler G et al., J Exp Med. 2004;200:123-135.
Kohler & Milstein, Nature. 1975;256:495.
Kohli-Kumar M et al., Blood. 84:2050-4,1994.
Krause DS, et al. Cell. 2001;105:369-377.
Kusadasi N et al., Leukemia. 2002;16:1782-1790.
Kyba M et al., Cell. 2002;109:29-37.
Kyba M et al., Proc Natl Acad Sci USA. 2003;100, Suppl 1:11904-11910.
Lagasse E, et al. Nat Med. 2000;6:1229-1234.
Lanier LL. "NK Cell Recognition." Annu Rev Immunol. 2004.
Laquerre, S., et al. J. Virol. 1998;72:9683-9697.
Larrick, et al., Methods: A Companion to Methods in Enzymology. (1991).
Lawrence, H. Blood 1997;89:1922.
Lefebvre V. Matrix Biol 1998;16:529-40.
Leung AYH et al., Dev Biol. 2004.
Lewis ID et al., Blood. 2001;97:3441-3449.
Lim, J.W. and Bodnar, A., Proteomics. 2002;2(9):1187-1203 (2002).
Liu HJ, Lamming C, C.M. V. Characterization of Human Bone Marrow and Umbilical Cord Blood Self-Renewing Multi-lineage Hematopoietic Stem Cells. 2003.
Liu JM: Fanconi's anemia, in Young NS (ed): Bone marrow failure syndromes. Philadelphia, W.B.Saunders company, 2000, p 47-68.
Loeffler, J. and Behr, J., Methods in Enzymology. 1993;217:599-618.
Marks et al., J. Mol Biol. 1991;222:581-597.
Martin, F., et al., J. Virol. 1999;73:6923-6929.
Matthew, H.W., et al., ASAIO Trans. 1991;37(3):M328-30.
Medvinsky A et al., Cell. 1996;86:897.
Mikkola HK et al., Blood. 2003;101:508-516.
Miller, A.D., and C. Buttimore, Mol. Cell. Biol. 1986;6:2895-2902.
Mochizuki, H., et al., J. Virol. 1998;72:8873-8883.
Molin, M., et al. J. Virol. 1998;72:8358-8361.
Morrison et al. Proc. Natl. Acad Sci. 1984;81, 6851-6855.
Muguruma Y et al., Exp Hematol. 2003;31:1323-1330.
Muschler, G.F., et al. J. Bone Joint Surg. Am. 1997;79(11):1699-709.
Nakano T et al., Science. 1994;265:1098-1101.
Nichols J et al., Cell. 1998;95:379-391.
Novotny and Haber, Proc. Natl. Acad. Sci. USA. 1985;82;4592-4596.
Offield, M.F., Development 1996;122:983-95.
Oostendorp RA et al., Blood. 2002;99:1183-1189.
Oostendorp RA et al., J Cell Sci. 2002;115:2099-2108.
Pack, et al., Bio/Technology. 1993;11:1271-77.
Packer, A.I., Dev Dyn 2000;17:62-74.
Persons, D., et al., Nature Medicine. 1998;4:1201-1205.
Petersen, Science. 1999;284:1168-1170.
Pittenger, Science 1999;284:143-147.
Potocnik AJ et al., Proc Natl Acad Sci USA. 1997;94:10295-10300.
Presta, Curr. Op. Struct. Biol. 1992;2:593-596.
Punzel M et al., Blood. 1999;93:3750-3756.
Rackoff WR et al., Blood. 1996;88:1588-93.
Rebel VI et al., Blood. 1996;87:3500-3507.
Reichmann et al., Nature. 1988;332:323-329.
Reya T et al., Nature. 2003;423:409-414.
Reyes M et al., Ann N Y Acad Sci. 2001;938:231-233; discussion 233-235.
Reyes M et al., Blood. 2001;98:2615-2625.
Reyes M et al., J Clin Invest. 2002;109:337-346.
Reyes M, and Verfaillie CM. Ann N Y Acad Sci. 2001;938:231-233; discussion 233-235.
Reyes M, et al., J Clin Invest. 2002;109:337-346.
Rideout WMr et al. Cell. 2002;109:17-27.
Robbins, et al. J. Virol. 1997;71(12):9466-9474.
Rosfjord E, Rizzino A. Biochem Biophys Res Commun. 1997;203:1795-802.
Rowley J. Cancer. 1990;65:2178-2184.
Salmons, B. and Gunzburg, W.H., 1993;4:129-141.
Scagni P et al., Haematologica 1988; 83:432-7.
Schuh AC, et al., Proc Natl Acad Sci USA. 1999;96:2159-2164.
Schwartz RE et al., J Clin Investigation. 2002;96:1291-1302.
Schwartz RE, et al. J Clin Invest. 2002;109:1291-1302.
Schwarzenberger, P., et al., J. Virol. 1997;71:8563-8571.
Sebestyen, et al. Nature Biotech. 1998;16:80-85.
Shimozaki et al. Development. 2003;130:2505-12.
Spangrude G et al., Science. 1988;241:58.
Sutton, R., et al., J. Virol. 1998;72:5781-5788.
Takahashi, J Clin Invest. 2000;105:71-7.
Takahashi, Nat Med. 1999;5:434-8.
Theise, Hepatology. 2000a;31:235-40.
Theise, Hepatology. 2000b;32:11-6.
Theunissen K and Verfaillie CM. Exp Hematol. 2004.
Thomas E et al., Ann Int Med. 1986;104:155-163.
Thomas ED. Semin Hematol. 1999;36:95-103.
Thomson JA et al., Science. 1998;282:1145-1147.
Tian X et al., Exp Hematol. 2004;32:1000-1009.
Tolar J et al., Blood. 2003; ASH Abstract.
Tolar J et al., Blood. 2004;ASH Abstract.
Traggiai E et al., Science. 2004;304:104-107.
Uwanogho D. et al., Mech Dev 1995; 49:23-36.
Vaswani, et al., Annals Allergy, Asthma & Immunol. 1998;81:105-115.
Verfaillie C et al., Blood. 1992;79:1003-1010.
Verfaillie C et al., Blood. 1998;92:1820-1831.
Verfaillie C et al., J Exp Med. 1991;174:693-703.
Verfaillie C. Blood. 1992;79:2821-2826.
Verfaillie CM et al., J Clin Invest. 1992;90:1232.
Verfaillie CM, et al., Blood. 1996;87:4770-4779.
Verfaillie, C.M. Trends Cell Biol. 2002;12(11):502-8.
Vodyanik MA et al., Blood. 2005;105:617-626.
Wagers AJ, et al. Science. 2002;297:2256-2259.
Wagner, E., et al., Proc. Natl. Acad. Sci. USA. 1992;89:6099-6103.
Wang L et al., Immunity. 2004;21:31-41.
Wang X et al., Nature. 2003;422:897-901.
Whitlow, et al., Methods: A Companion to Methods in Enzymology (1991).
Williams, R.S., et al., Proc. Natl. Acad. Sci. USA. 1991;88:2726-2730.
Wold, W., Adenovirus Methods and Protocols, Humana Methods in Molecular Medicine (1998), Blackwell Science, Ltd.
Wu GD, et al. Transplantation. 2003;75:679-685.
Wysocki and Sato, Proc. Natl. Acad. Sci. (USA). 1978;75:2844.
Xiong, C., et al., Science. 1989;243:1188-1191.
Yahata T et al., J Immunol. 2002;69:204-209.
Yanagi, K., et al., ASAIO Trans. 1989;35(3):570-2.
Yang, N.S., et al., Proc. Natl. Acad. Sci. USA. 1990;87:9568-9572.
Yoder M et al., Proc Natl Acad Sci USA. 1997;94:6776.
Zhang, G. et al., Biochem. Biophys. Res. Commun. 1996;227(3):707-711.
Zeng L et al., Blood. 2004; ASH abstract.
Zhao LR, et al. Exp Neurol. 2002;174:11-20.
Zhao R et al., Blood. 1997;90:4687-4698.
Zhao RCH et al., Blood. 2001;97:2406-2412.

All publications, patents and patent applications are incorporated herein by reference. While in the foregoing specification this invention has been described in relation to certain preferred embodiments thereof, and many details have been set forth for purposes of illustration, it will be apparent to those skilled in the art that the invention is susceptible to additional embodiments and that certain of the details described herein may be varied considerably without departing from the basic principles of the invention.

## Claims

1. Multipotent adult progenitor cells (MAPCs) that is human non-embryonic stem (ES), non-germ and non-embryonic germ cells that are positive for one or more of telomerase, oct-4, rex-1, rox-1, sox-2 or SSEA-4, that have been isolated and/or cultured in the presence of an oxygen concentration less than 10% for use in a method for the treatment of a congenital or acquired lymphohematopoietic disorder by providing lymphohematopoietic cells of lymphoid, myeloid and erythroid lineage, including T-lymphoid cells, in a tissue of the lymphoematopoietic system.

2. The cells for use according to claim 1 wherein the MAPCs can differentiate into cell types of three germ layers (ectodermal, endodermal and mesodermal).

3. The cells for use according to claim 1 or 2, wherein the oxygen concentration is from 3-5%.

4. The cells for use according to any one of claims 1-3, wherein the MAPCs express oct-4 and/or telomerase.

5. The cells for use according to claim 4, wherein the MAPCs express oct-4 and telomerase.

6. The cells for use according to any one of claims 1 to 5 for use with a Natural Killer cell function inhibitor.

7. The cells for use according to any one of claims 1 to 6, wherein the disorder comprises a leukemia, a myelodysplastic syndrome, a lymphoma, an inherited red blood cell abnormality, an anemia, an inherited platelet abnormality, an immune disorder, a lymphoproliferative disorder, a phagocyte disorder or a coagulation disorder.

8. The cells for use according to any one of claims 1 to 6, wherein the disorder comprises chronic mylogenous leukemia (CML).

9. The cells for use according to any one of claims 1 to 6, wherein the disorder comprises Fanconi's anemia.

10. The cells for use according to any claims 1-6 wherein the acquired lymphohematopoietic disorder has been induced by exposure to radiation or chemotherapy or is an inherited genetic deficiency.

11. The cells for use according to claim 7, wherein the anemia is sickle-cell anemia.

12. The cells for use according to claim 7, wherein the immune disorder is an inherited immune deficiency.

13. The cells use according to any of claims 1-6, wherein the MAPCs are autologous or allogeneic.

14. The cells for use according to any of one of claims 1-13, wherein the tissue is one or more of thymus, spleen, blood, bone marrow or lymph nodes.

## Patentansprüche

1. Multipotene adulte Vorläuferzellen (MAPCs), die humane nichtembryonische Stammzellen (ES), nicht-Keimzellen und nicht-embryonische Keimzellen sind, die für ein oder mehrere aus Telomerase, oct-4, rex-1, rox-1, sox-2 oder SSEA-4 positiv sind und die in Gegenwart von einer Sauerstoffkonzentration von weniger als 10% isoliert und/oder kultiviert worden sind, für die Verwendung in einem Verfahren zur Behandlung einer angeborenen oder erworbenen lymphohämatopoetischen Störung durch das Bereitstellen von lymphohämatopoetischen Zellen der lymphoiden, myeloiden und erythroiden Linie, einschließlich von T-lymphoiden Zellen, in einem Gewebe des lymphohämatopoetischen Systems.

2. Zellen zur Verwerdung nach Anspruch 1, wobei die MAPCs zu Zelltypen der drei Keimblätter differenzieren können (ektodermen, endodermem und mesodermen).

3. Zellen zur Verwerdung nach Anspruch 1 oder 2, wobei die Sauerstoffkonzentration 3 - 5 % ist.

4. Zellen zur Verwerdung nach einem der Ansprüche 1 - 3, wobei die MAPCs oct-4 und/oder Telomerase exprimieren.

5. Zellen zur Verwerdung nach Anspruch 4, wobei die MAPCs oct-4 und Telomerase exprimieren.

6. Zellen zur Verwerdung nach einem der Ansprüche 1 bis 5 zur Verwerdung mit einem Inhibitor für die natürliche Killerzellfunktion.

7. Zellen zur Verwerdung nach einem der Ansprüche 1 bis 6, wobei die Störung eine Leukämie, ein myelodysplastisches Syndrom, ein Lymphom, eine erbliche Anomalie der roten Blutkörperchen, eine Anämie, eine erbliche Blutplättchenanomalie, eine Immunstörung, eine lymphoproliferative Störung, eine Phagozytenstörung oder eine Koagulationsstörung umfasst.

8. Zellen zur Verwerdung nach einem der Ansprüche 1 bis 6, wobei die Störung eine chronische myeloische Leukämie (CML) umfasst.

9. Zellen zur Verwendung nach einem der Ansprüche 1 bis 6, wobei die Störung eine Fanconi-Anämie umfasst.

10. Zellen zur Verwerdung nach einem der Ansprüche 1 bis 6, wobei die erworbene lymphohämatopoetische Störung durch die Exposition zu einer Strahlung oder Chemotherapie induziert worden ist oder ein erbliches genetisches Defizit ist.

11. Zellen zur Verwerdung nach Anspruch 7, wobei die Anämie eine Sichelzellenanämie ist.

12. Zellen zur Verwerdung nach Anspruch 7, wobei die Immunstörrung ein erbliches Immundefizit ist.

13. Zellen zur Verwerdung nach Anspruch 1 - 6, wobei die MAPCs autolog oder allogen sind.

14. Zellen zur Verwerdung nach einem der Ansprüche 1 - 13, wobei das Gewebe eines oder mehrere aus Thymus, Milz, Blut, Knochenmark und Lymphknoten ist.

## Revendications

1. Cellules progénitrices adultes multipotentes (MAPC) qui sont des cellules souches non embryonnaires (ES) humaines, des cellules non germinales et germinales non embryonnaires qui sont positives concernant un ou plusieurs éléments parmi la télomérase, oct-4, rex-1, rox-1, sox-2 ou SSEA-4, qui ont été isolées et/ou mises en culture en présence d'une concentration en oxygène inférieure à 10 % pour une utilisation dans un procédé de traitement d'un trouble lymphohématopoïétique congénital ou acquis en fournissant des cellules lymphohématopoïétiques de lignée lymphoïde, myéloïde et érythroïde, incluant des cellules lymphoïdes T, dans un tissu du système lymphohématopoïétique.

2. Cellules à utiliser selon la revendication 1, dans lesquelles les MAPC peuvent se différencier en des types de cellules des trois couches germinales (ectodermique, endodermique et mésodermique).

3. Cellules à utiliser selon la revendication 1 ou 2, dans lesquelles la concentration en oxygène est de 3 à 5 %.

4. Cellules à utiliser selon l'une quelconque des revendications 1 à 3, dans lesquelles les MAPC expriment oct-4 et/ou la télomérase.

5. Cellules à utiliser selon la revendication 4, dans lesquelles les MAPC expriment oct-4 et la télomérase.

6. Cellules à utiliser selon l'une quelconque des revendications 1 à 5, pour une utilisation avec un inhibiteur de la fonction de cellule tueuse naturelle.

7. Cellules à utiliser selon l'une quelconque des revendications 1 à 6, dans lesquelles le trouble comprend une leucémie, un syndrome myélodysplasique, un lymphome, une anomalie héréditaire des globules rouges, une anémie, une anomalie plaquettaire héréditaire, un trouble immunitaire, un trouble lymphoprolifératif, un trouble phagocytaire ou un trouble de la coagulation.

8. Cellules à utiliser selon l'une quelconque des revendications 1 à 6, dans lesquelles le trouble comprend la leucémie myéloïde chronique (LMC).

9. Cellules à utiliser selon l'une quelconque des revendications 1 à 6, dans lesquelles le trouble comprend l'anémie de Fanconi.

10. Cellules à utiliser selon l'une quelconque des revendications 1 à 6, dans lesquelles le trouble lymphohématopoïétique acquis a été induit par une exposition à un rayonnement ou une chimiothérapie ou est une déficience génétique héréditaire.

11. Cellules à utiliser selon la revendication 7, dans lesquelles l'anémie est une anémie drépanocytaire.

12. Cellules à utiliser selon la revendication 7, dans lesquelles la déficience immunitaire est une déficience immunitaire héréditaire.

13. Cellules à utiliser selon l'une quelconque des revendications 1 à 6, dans lesquelles les MAPC sont autologues ou allogéniques.

14. Cellules à utiliser selon l'une quelconque des revendications 1 à 13, dans lesquelles le tissu est un ou plusieurs éléments parmi le thymus, la rate, le sang, la moelle osseuse ou les ganglions lymphatiques.
